# EUROPEAN PATENT SPECIFICATION

(11) **EP 2 133 384 B1**
(45) Date of publication and mention of the grant of the patent: **18.01.2012**
(21) Application number: 09251506.3
(22) Date of filing: 08.06.2009
(51) Int. Cl.: C08G 65/38, C07D 207/00, C08G 65/40, C08G 65/44, C08G 65/48

(54) **Bismaleamic acid, bismaleimide and cured product thereof**
Bismaleaminsäure, Bismaleinimid und gehärtetes Produkt davon
Acide bismaléamique, bismaleimide et produit durci associé

(30) Priority: 09.06.2008 JP 2008150517
(43) Date of publication of application: 16.12.2009
(73) Proprietor: Mitsubishi Gas Chemical Company, Inc., Tokyo (JP)
(72) Inventor: Uera, Kazuyoshi, Katsushika-ku Tokyo (JP); Ohno, Saisuke, Katsushika-ku Tokyo (JP); Ishii, Kenji, Tokyo (JP)
(74) Representative: McCluskie, Gail Wilson

(56) References cited:
- JP-A- 4 261 411
- JP-A- 7 215 933
- JP-A- 2002 294 064
- JP-A- 2003 277 360
- JP-A- 2005 125 677

## Description

### Technical Field of the Invention

The present invention relates to a bismaleamic acid obtained by reacting an aromatic diamine, obtained from as a raw material a bifunctional phenylene ether oligomer having a specific structure, with maleic anhydride, a bismaleimide and processes for the production of these and to a curable resin composition containing the above bismaleimide and a cured product thereof.

### Background Art of the Invention

A bismaleimide resin is generally known as a highly heat-resistant thermosetting resin and widely used in the fields of molding materials, composite materials and electrical and electronic parts. In recent years, developments of microelectronics technologies are remarkable in the field of electrical and electronic parts. In particular, large computers employ multilayer circuit boards so that high-speed signal transmission is necessary to the large computers. However, when the dielectric constant of a board material is large, a delay in signal transmission occurs, which hinders speed-up. A bismaleimide is used for an interlayer insulation film of a multilayer wiring structure. From the above reasons, attention is riveted to the need of a bismaleimide maintaining heat resistance and also having a low dielectric constant.

On the other hand, for the purpose of securing insulation reliability and dimensional stability, which are essential for high-density packaging accompanied by the recent progress of electronic materials, a resin for an electronic material is required to be free from a change in dielectric characteristics even in high humidity and also free from a dimensional change such as swelling or expansion and contraction. However, generally, the dielectric characteristics of a bismaleimide at a moisture-absorbed time largely differ from those at a dried time since an imide group is apt to absorb moisture. This is a problem with regard to the employment of a bismaleimide as an electronic material. Furthermore, a bismaleimide is used in the form of a varnish in such electronic material applications in many cases so that excellent solvent solubility is required in terms of workability.

A variety of bismaleimides and bismaleamic acids, which are raw materials for bismaleimides, have been proposed for solving these problems (for instance, JP-A-4-261411 and JP-A-7-215933). However, these proposals are not sufficient for coping with the recent demand for further high-performance in the electronic material field.

### Summary of the Invention

It is an object of the present invention to provide a bismaleimide useful as a high molecular weight material having high heat resistance, a low dielectric constant, a low water absorption coefficient and excellent solvent solubility, a bismaleamic acid capable of generating the above bismaleimide, a resin composition containing the above bismaleimide and a cured product obtained by curing the above resin composition.

The present inventors have developed bifunctional phenylene ether oligomers having specific structures, which oligomers have the excellent low dielectric characteristics and heat resistance of a polyphenylene ether structure, and their derivatives. The present inventors have made further diligent studies and as a result found that a terminal aromatic diamine can be derived from a bifunctional phenylene ether oligomer through a terminal bifunctional phenylene ether oligomer dinitro compound and that a bismaleimide which is excellent in solvent solubility, is capable of giving a cured product having high heat resistance and low dielectric characteristics and is almost free from a change in dielectric characteristics even in high humidity can be obtained from the above terminal aromatic diamine and maleic anhydride. On the basis of the above findings, the present inventors have completed the present invention.

The present invention provides a bismaleimide represented by the formula (1), 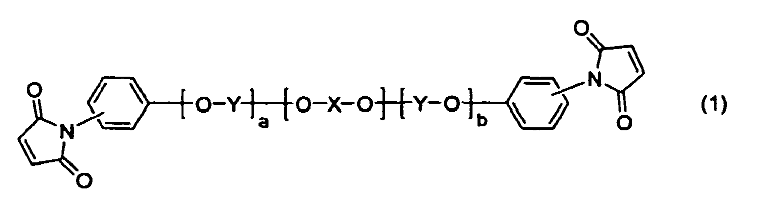 wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each imide group is a para-position or a meta-position, 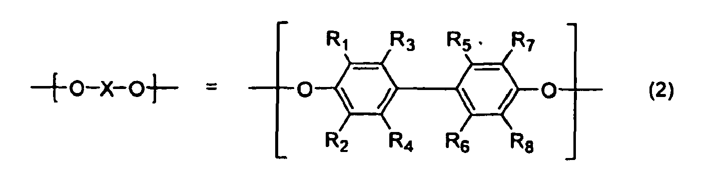 wherein R₁, R₂, R₃, R₇ and R₈ are the same or different and represent a halogen atom, an alkyl group having 6 or less carbon atoms or a phenyl group, R₄, R₅ and R₆ are the same or different and represent a hydrogen atom, a halogen atom, an alkyl group having 6 or less carbon atoms or a phenyl group, 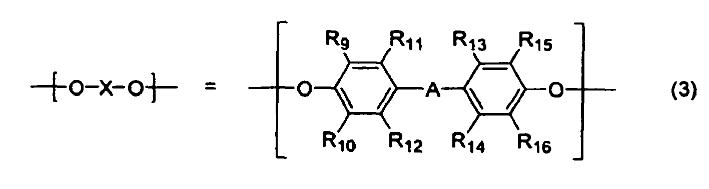 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- is a linear, branched or cyclic bivalent hydrocarbon group having 20 or less carbon atoms, 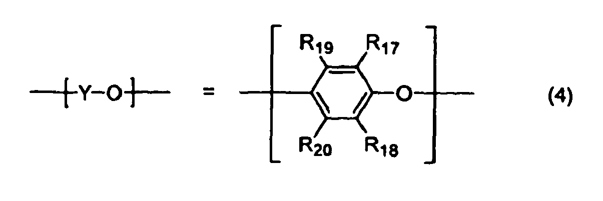 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

The present invention further provides a bismaleamic acid represented by the formula (10), 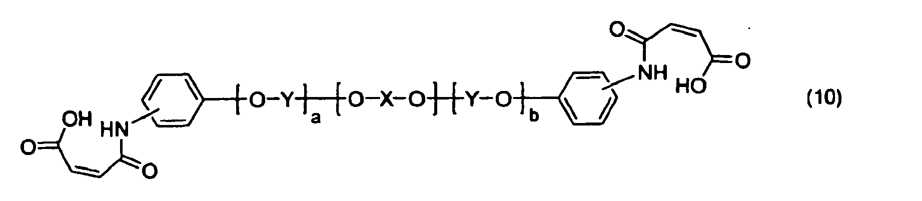 wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each amide group is a para-position or a meta-position.

The present invention furthermore provides a process for the production of the bismaleamic acid represented by the formula (10), which process comprises reacting a diamine represented by the formula (11) with maleic anhydride, 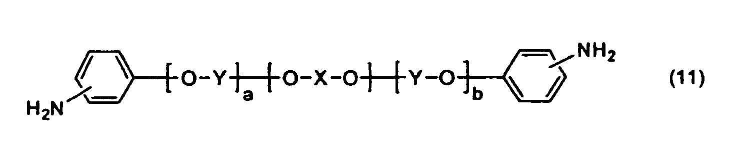 wherein -(O-X-O)-, -(Y-O)-, a and b are as defined in the formula (10).

The present invention still further provides a process for the production of the bismaleimide represented by the formula (1), which process comprises cyclodehydrating and imidizing the bismaleamic acid represented by the formula (10).

The present invention moreover provides a curable resin composition containing at least one kind of bismaleimide represented by the formula (1) and a cured product obtained by curing at least one kind of bismaleimide represented by the formula (1).

### Effect of the Invention

The bismaleamic acid provided by the present invention can easily generates a bismaleimide excellent in low dielectric characteristics, heat resistance, low water absorptivity and solvent solubility. The resin composition comprising the bismaleimide provided by the present invention exhibits only a small change in dielectric characteristics in high humidity so that it is usable for wide applications such as a resin for a copper-clad laminate, a resin for a resist, a sealing resin for electronic parts, a resin for a color filter of a liquid crystal, a coating, a variety of coating agents, an adhesive, a buildup laminate material, a resin for a flexible substrate and a functional film.

### Brief Description of Drawings

Fig.1 shows IR spectrum of a resin "C" in Synthetic Example 1.
Fig.2 shows ¹H NMR spectrum of a resin "C" in Synthetic Example 1.
Fig.3 shows FD mass spectrum of a resin "C" in Synthetic Example 1.
Fig.4 shows IR spectrum of a resin "S" in Example 1.
Fig.5 shows IR spectrum of a resin "Y" in Example 2.
Fig.6 shows ¹H NMR spectrum of a resin "Y" in Example 2.
Fig.7 shows FD mass spectrum of a resin "Y" in Example 2.

### Detailed Description of the Invention

The present invention will be explained in detail hereinafter. First, in the bismaleamic acid represented by the formula (10), -(O-X-O)- represents a structure defined by the formula (2) or the formula (3), -(Y-O)- represents an arrangement of one kind of structure defined by the formula (4) or a random arrangement of at least two kinds of structures defined by the formula (4) and each of a and b is an integer of 0 to 100, provided that at least one of a and b is not 0. In the formula (2), R₁, R₂, R₃, R₇ and R₈ are the same or different and represent a halogen atom, an alkyl group having 6 or less carbon atoms or a phenyl group and R₄, R₅ and R₆ are the same or different and represent a hydrogen atom, a halogen atom, an alkyl group having 6 or less carbon atoms or a phenyl group. In the formula (3), R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms. In the formula (4), R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

In particular, preferred are a bismaleamic acid represented by the formula (10) wherein R₁, R₂, R₃, R₇ and R₈ in the formula (2) represent an alkyl group having 3 or less carbon atoms and R₄, R₅ and R₆ in the formula (2) represent a hydrogen atom or an alkyl group having 3 or less carbon atoms, a bismaleamic acid represented by the formula (10) wherein R₉, R₁₀, R₁₁, R₁₂, R₁₃, R₁₄, R₁₅ and R₁₆ in the formula (3) represent a hydrogen atom or an alkyl group having 3 or less carbon atoms and a bismaleamic acid represented by the formula (10) wherein R₁₇ and R₁₈ in the formula (4) represent an alkyl group having 3 or less carbon atoms and R₁₉ and R₂₀ in the formula (4) represent a hydrogen atom or an alkyl group having 3 or less carbon atoms. Especially preferred is a bismaleamic acid represented by the formula (10) wherein -(O-X-O)- represented by the formula (2) is a structure represented by the formula (5) or -(O-X-O)- represented by the formula (3) is a structure represented by the formula (6) or the formula (7) and -(Y-O)- represented by the formula (4) is an arrangement of a structure of the formula (8) or the formula (9) or a random arrangement of a structure of the formula (8) and a structure of the formula (9).

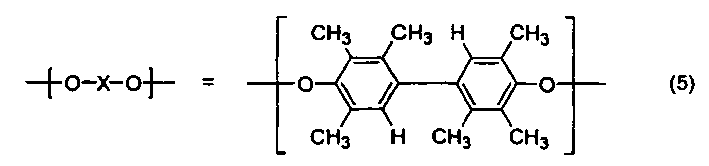

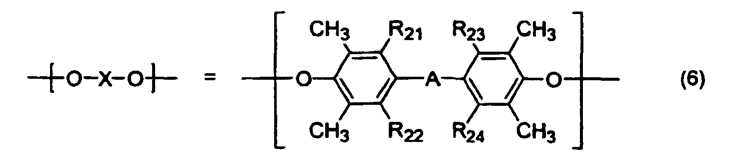

wherein R₂₁, R₂₂, R₂₃ and R₂₄ are the same or different and represent a hydrogen atom or a methyl group and -A- is a linear, branched or cyclic bivalent hydrocarbon group having 20 or less carbon atoms.

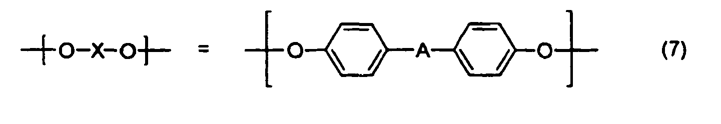

wherein -A- is a linear, branched or cyclic bivalent hydrocarbon group having 20 or less carbon atoms.

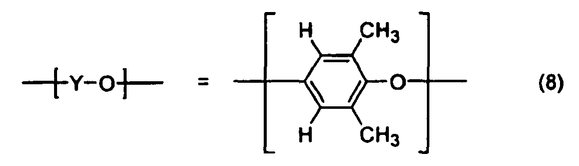

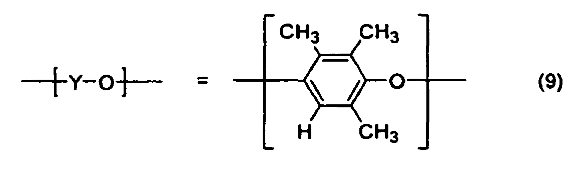

Examples of -A- in the formula (3) include bivalent hydrocarbon groups such as methylene, ethylidene, 1-methylethylidene, 1,1-propylidene, 1,4-phenylenebis(1-methylethylidene), 1,3-phenylenebis(1-methylethylidene), cyclohexylidene, phenylmethylene, naphthylmethylene and 1-phenylethylidene. -Ain the formula (3) is not limited to these examples.

The bismaleamic acid represented by the formula (10) , provided by the present invention, can be produced by reacting a bifunctional phenylene ether oligomer diamine represented by the formula (11) with maleic anhydride in an organic solvent.

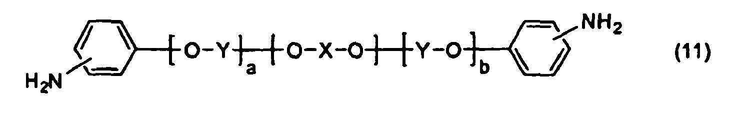

wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each amide group is a para-position or a meta-position, 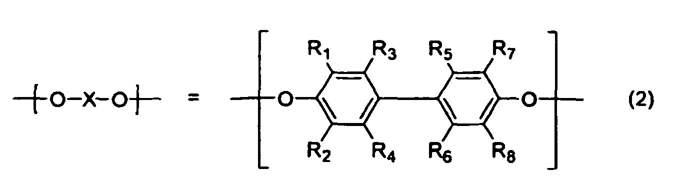 wherein R₁, R₂, R₃, R₇ and R₈ are the same or different and represent a halogen atom, an alkyl group having 6 or less carbon atoms or a phenyl group, R₄, R₅ and R₆ are the same or different and represent a hydrogen atom, a halogen atom, an alkyl group having 6 or less carbon atoms or a phenyl group, 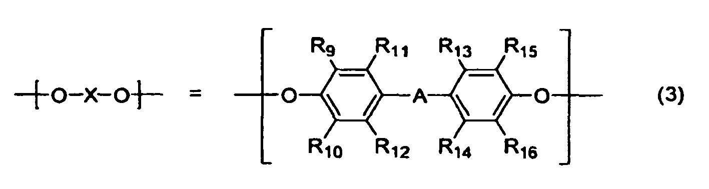 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- is a linear, branched or cyclic bivalent hydrocarbon group having 20 or less carbon atoms, 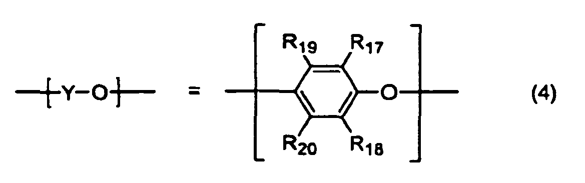 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

Examples of the organic solvent include N,N-dimethylformamide, N,N-dimethylacetamide, N,N-dimethylmethoxyacetamide, N,N-diethylmethoxyacetamide, N-methyl-2-pyrrolidone, N-methylcaprolactam, 1,2-dimethoxyethane, diethylene glycol dimethyl ether, diethylene glycol ethyl methyl ether, diethylene glycol diethyl ether, tetrahydrofuran, 1,3-dioxane, 1,4-dioxane, pyridine, picoline, dimethylsulfoxide, dimethylsulfone, tetramethylurea, ethyl acetate, methyl acetate, 2-butanone, toluene, xylene and mesitylene. The organic solvent can be used singly or at least two organic solvents can be used in combination. The concentration of the reaction raw materials is generally 2 to 50 wt%, preferably 5 to 30 wt%. The reaction temperature is generally 60 °C or lower, preferably 50 °C or lower. The reaction pressure is not specially limited. Generally, the reaction can be carried out under normal pressure. Further, the reaction time is generally 0.5 to 24 hours. The bismaleamic acid of the present invention can be obtained by the above-explained reaction.

The method for producing the bifunctional phenylene ether oligomer diamine used for the synthesis of the bismaleamic acid of the present invention is not specially limited. The bifunctional phenylene ether oligomer diamine can be produced by any method. For example, it can be produced by reducing a bifunctional phenylene ether oligomer dinitro compound obtained by reacting a bifunctional phenylene ether oligomer, which is obtained by oxidatively coupling a bifunctional phenol compound and a monofunctional phenol compound, with a nitro halobenzene compound or a dinitro benzene compound in an organic solvent in the presence of a basic compound.

The method of the above reduction of the bifunctional phenylene ether oligomer dinitro compound is not specially limited. For example, it is possible to adopt a known method in which a nitro group is reduced to an amino group. The reduction reaction of the bifunctional phenylene ether oligomer dinitro compound is carried out by, for example, reducing the bifunctional phenylene ether oligomer dinitro compound to an bifunctional phenylene ether oligomer diamino compound by use of hydrogen in a reaction solvent, which is inactive in the reaction, at a temperature of 20 to 200°C at a pressure of normal pressure to 50kgf/cm² in the presence of a hydrogenation catalyst such as a metal catalyst typified by nickel, palladium or platinum, a supported catalyst in which such a metal is carried on a proper support, or a Raney catalyst of nickel, copper or the like. Examples of the above reaction solvent include aliphatic alcohols such as methanol, ethanol and isopropanol, ethylene glycol monoalkyl ethers such as methyl cellosolve and ethyl cellosolve, aromatic hydrocarbons such as toluene, benzene and xylene, and ethers such as tetrahydrofuran, dioxane, dipropyl ether, diethylene glycol dimethyl ether, diethylene glycol ethyl methyl ether and diethylene glycol diethyl ether. The reaction solvent is not limited to these examples so long as it is a solvent which dissolves the bifunctional phenylene ether oligomer dinitro compound. The reaction solvent may be used singly or at least two reaction solvents may be used in combination.

The number average molecular weight of the bifunctional phenylene ether oligomer diamine used for the synthesis of the bismaleamic acid of the present invention is preferably in the range of from 500 to 3,000. The molecular weight distribution (weight average molecular weight/number average molecular weight) is preferably in the range of 1 to 3. When the number average molecular weight is less than 500, it is difficult to obtain electric characteristics possessed by a phenylene ether structure. When it exceeds 3,000, the reactivity of a terminal functional group is decreased and the solubility into solvent is also decreased. When the molecular weight distribution exceeds 3, the solubility into solvent is decreased.

The substitution position of an amino group of the bifunctional phenylene ether oligomer diamine used for the synthesis of the bismaleamic acid of the present invention is not specially limited so long as it is a para-position or a meta-position.

Then, the bifunctional phenylene ether oligomer dinitro compound, which can be a precursor of the above bifunctional phenylene ether oligomer diamine, will be explained. The method for producing the bifunctional phenylene ether oligomer dinitro compound is not specially limited. The bifunctional phenylene ether oligomer dinitro compound can be produced by any method. For example, it is produced by reacting a bifunctional phenylene ether oligomer, which is obtained by oxidatively coupling a bifunctional phenol compound and a monofunctional phenol compound, with a nitro halobenzene compound or a dinitro benzene compound in an organic solvent in the presence of a basic compound at a temperature of 50 to 250°C, preferably 50 to 180°C, for 0.5 to 24 hours. The method for producing the bifunctional phenylene ether oligomer dinitro compound can be selected from known methods. For example, the methods described in JP-A-4-178358, JP-A-2006-219396, etc., can be adopted.

The above-mentioned bifunctional phenylene ether oligomer can be produced by, for example, dissolving a bifunctional phenol compound, a monofunctional phenol compound and a catalyst in a solvent and then introducing oxygen into the resultant solution under heat with stirring. Examples of the bifunctional phenol compound include 2,2',3,3',5,5'-hexamethyl-(1,1'-biphenyl)-4,4'-diol, 4,4'-methylenebis(2,6-dimethylphenol), bis(4-hydroxyphenyl)methane and 2,2-bis (4-hydroxyphenyl) propane. The bifunctional phenol compound is not limited to these examples. The monofunctional phenol compound is typically 2,6-dimethylphenol or 2,3,6-trimethylphenol. The monofunctional phenol compound is not limited to these. The catalyst is, for example, a combination of a copper salt and an amine. Examples of the copper salt include CuCl, CuBr, CuI, CuCl₂ and CuBr₂. Examples of the amine include di-n-butylamine, n-butyldimethylamine, N,N'-di-t-butylethylenediamine, pyridine, N,N,N'N'-tetramethylethylenediamine, piperidine and imidazole. The catalyst is not limited to these examples. Examples of the solvent include toluene, methanol, methyl ethyl ketone and xylene. The solvent is not limited to these examples.

Then, the bismaleimide of the present invention will be explained. In the bismaleimide represented by the formula (1), -(O-X-O)- represents a structure defined by the formula (2) or the formula (3), -(Y-O)- represents an arrangement of one kind of structure defined by the formula (4) or a random arrangement of at least two kinds of structures defined by the formula (4) and each of a and b is an integer of 0 to 100, provided that at least one of a and b is not 0. In the formula (2), R₁, R₂, R₃, R₇ and R₈ are the same or different and represent a halogen atom, an alkyl group having 6 or less carbon atoms or a phenyl group and R₄, R₅ and R₆ are the same or different and represent a hydrogen atom, a halogen atom, an alkyl group having 6 or less carbon atoms or a phenyl group. In the formula (3), R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms. In the formula (4), R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

In particular, preferred are a bismaleimide represented by the formula (1) wherein R₁, R₂, R₃, R₇ and R₈ in the formula (2) represent an alkyl group having 3 or less carbon atoms and R₄, R₅ and R₆ in the formula (2) represent a hydrogen atom or an alkyl group having 3 or less carbon atoms, a bismaleimide represented by the formula (1) wherein R₉, R₁₀, R₁₁, R₁₂, R₁₃, R₁₄, R₁₅ and R₁₆ in the formula (3) represent a hydrogen atom or an alkyl group having 3 or less carbon atoms and a bismaleimide represented by the formula (1) wherein R₁₇ and R₁₈ in the formula (4) represent an alkyl group having 3 or less carbon atoms and R₁₉ and R₂₀ in the formula (4) represent a hydrogen atom or an alkyl group having 3 or less carbon atoms. Especially preferred is a bismaleimide represented by the formula (1) wherein -(O-X-O)- represented by the formula (2) is a structure represented by the formula (5) or -(O-X-O)- represented by the formula (3) is a structure represented by the formula (6) or the formula (7) and -(Y-O)- represented by the formula (4) is an arrangement of a structure of the formula (8) or the formula (9) or a random arrangement of a structure of the formula (8) and a structure of the formula (9).

The bismaleimide of the present invention can be produced by imidizing the bismaleamic acid of the formula (10) through cyclodehydration.

The method for the above imidation can be selected from known methods such as a method in which a dehydrating agent such as acetic anhydride and a catalyst are added (for instance, JP-A-4-261411) or a method in which the bismaleamic acid is cyclodehydrated by heating it in an aromatic hydrocarbon solvent such as toluene or xylene in the presence of an acid catalyst to carry out imidation (for instance, JP-A-7-118230). The imidation method is not limited to these methods.

Examples of a solvent used for the above imidation reaction comprising the addition of the dehydrating agent include fatty acid esters such as butyl formate, isobutyl formate, t-butyl formate, ethyl acetate, propyl acetate, isopropyl acetate, butyl acetate, isobutyl acetate, t-butyl acetate, methyl propionate and ethyl propionate, ethylene glycol monoalkyl ethers such as methyl cellosolve and ethyl cellosolve, and ethers such as tetrahydrofuran, dioxane, dipropyl ether, diethylene glycol dimethyl ether, diethylene glycol ethyl methyl ether and diethylene glycol diethyl ether. The solvent is not limited to these examples so long as it dissolves the bismaleamic acid of the present invention. Further, the above reaction solvent can be used singly or at least two solvents can be used in combination.

A carboxylic anhydride used as the dehydrating agent is virtually limited to acetic anhydride from economic reasons. However, it is not specially limited. Further, a carbonate, a hydrogen carbonate, a sulfate, a nitrate, a phosphate, an acetate, a formate or a higher fatty acid salt of an alkali metal (preferably, sodium, potassium or lithium) or a salt or a halide of a transition metal such as nickel, cobalt or manganese is used as the catalyst used for the cyclodehydration. Potassium acetate is preferred. The total amount of the dehydrating agent and the catalyst is preferably 0.1 to 4.0 mol based on 1 mol of an amic acid group. Further, a tertiary amine or a pyridine derivative can be added, as required, in order to accelerate the reaction in the cyclodehydration reaction. Examples of the tertiary amine and the pyridine derivative include triethylamine, tripropylamine, tributylamine, triphenylamine, pyridine, methyl pyridine and 2,6-dimethylpyridine. The amount of the above reaction accelerator is approximately 0.1 to 2.0 mol based on 1 mol of an amic acid group.

The imidation is carried out by carrying out the reaction at a reaction temperature of 0 to 60 °C, preferably 10 to 40°C, for 0.5 to 100 hours.

The solvent used in the aforesaid imidation reaction in which heating is carried out in the presence of the acid catalyst include aromatic hydrocarbon solvents such as benzene, toluene, xylene and ethyl benzene. An aprotic polar solvent such as N,N-dimethylformamide, N,N-dimethylacetamide, N-methyl-2-pyrrolidone or dimethylsulfoxide can be added as required. The amount of the aprotic polar solvent in the reaction solvent is 0.05 to 20 % by weight, preferably 1 to 30 % by weight. The cyclodehydration is carried out at a temperature between 100 and 200 °C while water to be generated is removed by azeotropy, whereby the bismaleimide is obtained.

Examples of the acid catalyst used for the cyclodehydration reaction includes phosphoric acid, polyphosphoric acid, sulfuric acid and p-toluenesulfonic acid. The amount of the acid catalyst to be used is 0.001 to 0.1 mol, preferably 0.005 to 0.05 mol, based on 1 mol of an amic acid group.

Further, it is possible to adopt a method in which the aforementioned bismaleamic acid, obtained by reacting a bifunctional phenylene ether oligomer diamine with maleic anhydride, is isolated and then the bismaleamic acid is imidized or a method in which imidation is directly carried out without isolating the bismaleamic acid.

Then, the curable resin composition provided by the present invention will be explained. The above curable resin composition is characterized in that it contains one kind or at least two kinds of above-explained bismaleimide(s) of the present invention. The curable resin composition of the present invention can further contain a known epoxy resin, an oxetane resin, a compound having a polymerizable unsaturated group, a cyanate ester compound, a benzoxazine resin, photo-polymerizationand/or thermal polymerization initiators, a photosensitizer, a curing agent and the like.

The epoxy resin can be selected from generally known epoxy resins. Examples thereof include a bisphenol A type epoxy resin, a bisphenol F type epoxy resin, a biphenyl type epoxy resin, a phenol novolak type epoxy resin, a cresol novolak type epoxy resin, a xylene novolak type epoxy resin, triglycidyl isocyanurate, an alicyclic epoxy resin, a dicyclopentadiene novolak type epoxy resin, a biphenyl novolak type epoxy resin and epoxy resins having PPE structure disclosed in JP-A-2003-155340 and JP-A-2003-212990. The epoxy resin can be used singly or at least two epoxy resins can be used in combination.

The oxetane resin can be selected from generally known oxetane resins. Examples thereof include alkyl oxetanes such as oxetane, 2-methyloxetane, 2,2-dimethyloxetane, 3-methyloxetane and 3,3-dimethyloxetane, 3-methyl-3-methoxymethyloxetane, 3,3'-di(trifluoromethyl) perfluorooxetane, 2-chloromethyloxetane, 3,3-bis(chrolomethyl)oxetane, OXT-101 (trade name, supplied by TOAGOSEI Co., Ltd.) and OXT-121 (trade name, supplied by TOAGOSEI Co., Ltd.). The oxetane can be used singly or at least two oxetane resins can be used in combination.

When the epoxy resin and/or the oxetane resin are used in the curable resin composition of the present invention, an epoxy resin curing agent and/or an oxetane resin curing agent can be used. The epoxy resin curing agent is selected from generally known curing agents. Examples of the epoxy resin curing agent include imidazole derivatives such as 2-methylimidazole, 2-ethyl-4-methylimidazole, 2-phenylimidazole, 1-cyanoethyl-2-phenylimidazole, 1-cyanoethyl-2-ethyl-4-methylimidazole, 2-phenyl-4,5-dihydroxymethylimidazole and 2-phenyl-4-methyl-5-hydroxymethylimidazole; amine compounds such as dicyandiamide, benzyldimethylamine and 4-methyl-N,N-dimethylbenzylamine; and phosphine compounds and phosphonium compounds. A known cationic polymerization initiator can be used as the oxetane resin curing agent. Commercially available examples thereof include SAN-AID SI-60L, SAN-AID SI-80L, SAN-AID SI-100L (supplied by Sanshin Chemical Industry Co., Ltd.), CI-2064 (supplied by Nippon Soda Co., Ltd.), IRGACURE261 (supplied by Ciba Specialty Chemicals), ADEKAOPTMER SP-170, ADEKAOPTMER SP-150, (supplied by Asahi Denka Kogyo K.K.), and CYRACURE UVI-6990 (supplied by Union Carbide Corporation). The cationic polymerization initiator can be used also as an epoxy resin curing agent. The epoxy resin curing agent or the oxetane resin curing agent can be used singly or at least two epoxy resin curing agents or the oxetane resin curing agents can be used in combination.

The compound having a polymerizable unsaturated group can be selected from generally known compounds having a polymerizable unsaturated group. Examples thereof include vinyl compounds such as ethylene, propylene, styrene and vinyl compounds having PPE structure disclosed in JP-A-2004-59644, (meth)acrylates of monohydric and polyhydric alcohols such as methyl(meth)acrylate, 2-hydroxyethyl(meth)acrylate, 2-hydroxypropyl(meth)acrylate, polypropylene glycol di(meth)acrylate, trimethylol propane di(meth)acrylate, trimethylol propane tri(meth)acrylate, pentaerythritol tetra(meth)acrylate and dipentaerythritol hexa(meth)acrylate, epoxy (meth)acrylates such as bisphenol A type epoxy (meth)acrylate, bisphenol F type epoxy (meth)acrylate and epoxy (meth)acrylates having PPE structure disclosed in JP-A-2003-183350 and JP-A-2003-238653, (meth)acrylates having PPE structure disclosed in JP-A-2003-252983 and JP-A-2003-252833, a benzocyclobutene resin, triallyl isocyanurate (TAIC) and triallyl cyanurate(TAC). The compound having a polymerizable unsaturated group can be used singly or at least two compounds having a polymerizable unsaturated group can be used in combination.

The cyanate ester compound can be selected from generally known cyanate ester compounds. Examples thereof include bisphenol A dicyanate ester, bisphenol F dicyanate ester, bisphenol M dicyanate ester, bisphenol P dicyanate ester, bisphenol E dicyanate ester, phenol novolak type cyanate ester, cresol novolak type cyanate ester, dicyclopentadiene novolak type cyanate ester, tetramethyl bisphenol F dicyanate ester, biphenol dicyanate ester and cyanate esters disclosed in JP-A-2006-328286. The cyanate ester compound can be used singly or at least two cyanate ester compounds can be used in combination.

A known curing catalyst can be used for curing the cyanate ester compound. Examples thereof include metal salts such as zinc octylate, zinc naphthenate, cobalt naphthenate, copper naphthenate and acetylacetone iron, and compounds having an active hydroxyl group such as phenol, alcohol and amine.

The photo-polymerization initiator can be selected from generally known photo-polymerization initiators. Examples thereof include α-diketones such as benzyl and diacetyl, acyloin ethers such as benzoyl ethyl ether and benzoin isopropyl ether, thioxanthones such as thioxanthone, 2,4-diethylthioxanthone and 2-isopropylthioxanthone, benzophenones such as benzophenone and 4,4'-bis(dimethylamino)benzophenone, acetophenones such as acetophenone, 2,2'-dimethoxy-2-phenylacetophenone and β-methoxy acetophenone, and aminoacetophenones such as 2-methyl-1-[4-(methylthio)phenyl]-2-morpholinopropane-1-one and 2-benzyl-2-dimethylamino-1-(-4-morpholinophenyl)- butanone-1. The photo-polymerization initiator can be used singly or at least two photo-polymerization initiators can be used in combination.

Further, one kind of or at least two kinds of known photosensitizer(s) can be used in combination with the photo-polymerization initiator. Examples of the photosensitizer include N,N-dimethylaminobenzoic acid ethyl ester, N,N-dimethylaminobenzoic acid isoamyl ester, triethanolamine and triethylamine.

The thermal polymerization initiator can be selected from generally known thermal polymerization initiators. Examples thereof include peroxides such as benzoyl peroxide, parachlorobenzoyl peroxide, 2,4-dichlorobenzoyl peroxide, lauroyl peroxide, dicumyl peroxide, acetyl peroxide, methyl ethyl ketone peroxide, cyclohexanone peroxide, bis(1-hydroxycyclohexylperoxide), 2,5-dimethylhexane-2,5-dihydroxyperoxide, t-butylperbenzoate, 2,5-dimethyl-2,5-di(t-butylperoxy)hexane, 2,5-dimethyl-2,5-(t-butylperoxy)hexyne-3, 2,5-dimethylhexyl-2,5-di(peroxybenzoate), cumene hydroperoxide, t-butylhydroperoxide, t-butylperoxybenzoate, t-butylperoxyacetate, t-butylperoxyoctate, t-butylperoxyisobutylate, dibenzyl peroxide, di-t-butylperoxyphthalate, diisopropyl peroxycarbonate and di-2-ethylhexylperoxycarbonate, and azo compounds such as azobisisobutylonitrile. The thermal polymerization initiator can be used singly or at least two thermal polymerization initiators can be used in combination.

The amount of the polymerization initiator is 0.01 to 10 parts by weight, especially preferably 0.1 to 5 parts by weight, based on 100 parts by weight of the resin composition. A polymerization promoter, a delaying agent, a pigment and a filler can be added as required.

Further, a known polymerization inhibitor can be incorporated for the purpose of adjusting a curing degree. Examples thereof include quinones such as hydroquinone, methyl hydroquinone, t-butylhydroquinone, p-benzoquinone, chloranil and trimethyl quinone, aromatic diols and copper salts. The polymerization inhibitor can be used singly or at least two polymerization inhibitors can be used in combination.

An amine compound having at least two amino groups is used as the curing agent. Polymerization reaction proceeds by Michael addition of an amino group and a double bond of an imide ring of the bismaleimide compound. Examples of such amine compound include aromatic diamines such as diaminodiphenyl methane, diaminodiphenyl sulfone, phenylene diamine and xylene diamine, and halogenated derivatives of these.

A known additive can be added, as required, in the production of the curable resin composition of the present invention. Examples of the additive include a coupling agent, a thermoplastic resin, an inorganic filler, a color pigment, an antifoaming agent, a surface conditioner, a flame retardant, a ultraviolet absorber, an antioxidant and a fluidity regulator.

Examples of the coupling agent include silane coupling agents such as vinyltrichlorosilane, vinyltriethoxysilane, vinyltrimethoxysilane, γ-methacryloxypropyltrimethoxysilane, β(3,4epoxycyclohexyl)ethyltrimethoxysilane, γ-glycidoxypropylmethyldiethoxysilane, N-β(aminoethyl)γ-aminopropylmethylmethoxysilane, γ-aminopropyltriethoxysilane, N-phenyl-γ-aminopropyltrimethoxysilane, γ-mercaptopropyltrimethoxysilane and γ-chloropropyltrimethoxysilane, a titanate coupling agent, an aluminum coupling agent, a zircoaluminate coupling agent, a silicon coupling agent and a fluorine coupling agent. The coupling agent can be used singly or at least two coupling agents can be used in combination.

Examples of the thermoplastic resin include polyamidoimide, polyimide, polybutadiene, polyethylene, polystyrene, polycarbonate, a phenoxy resin, polyisoprene, polyester, polyvinyl butyral and polybutadiene.

Examples of the inorganic filler include silicas such as natural silica, fused silica and amorphous silica, white carbon, titanium white, Aerosil, alumina, talc, natural mica, synthetic mica, kaolin, clay, aluminum hydroxide, a heat-treated product of aluminum hydroxide (obtained by heat-treating aluminum hydroxide and reducing crystal water partially), boehmite, a metal hydrate such as magnesium hydroxide, barium sulfate, E-glass, A-glass, NE-glass, C-glass, L-glass, D-glass, S-glass and M-glass G20.

The flame retardant can be selected from known flame retardants. Examples thereof include halogen flame retardants such as a brominated epoxy resin, brominated polycarbonate, brominated polystyrene, brominated styrene, brominated maleimide, brominated phthalimide, tetrabromobisphenol A, pentabenzyl(meth)acrylate, pentabromotoluene, tribromophenol, hexabromobenzene, decabromodiphenyl ether, bis-1,2-pentabromophenylethane, chlorinated polystyrene and chlorinated paraffin; phosphoric flame retardants such as red phosphorus, tricresyl phosphate, triphenyl phosphate, cresyl diphenyl phosphate, trixylenyl phosphate, trialkyl phosphate, dialkyl phosphate, tris(chloroethyl)phosphate, phosphazene, 1,3-phenylenebis(2,6-dixylenylphosphate) and 10-(2,5-dihydroxyphenyl)-10H-9-oxa-10-phosphaphenanthrene -10-oxide; inorganic flame retardants such as aluminum hydroxide, magnesium hydroxide, boehmite, partial boehmite, zinc borate and antimony trioxide; and silicon flame retardants such as silicon rubber and a silicon resin. The flame retardant can be used singly or at least two flame retardants can be used in combination.

The antioxidant is selected from known antioxidants. Examples thereof include phenol antioxidants such as 2,6-di-t-butyl-4-methylphenol, 2,2'-methylenebis(6-t-butyl-4-methylphenol), N,N'-hexane-1,6-diylbis[3-(3,5-di-t-butyl-4-hydroxyphenyl) propionamide] and 2,6-di-t-butyl-4-(4,6-bis(octylthio)- 1,3,5-triazine-2-ylaminophenol, an amine antioxidant and a sulfur antioxidant. The antioxidant can be used singly or at least two antioxidants can be used in combination.

The curable resin composition of the present invention can be used, for example, as a prepreg which is obtained by dissolving the curable resin composition into a solvent, impregnating the thus-obtained solution into a glass cloth, an aramid nonwoven fabric, a liquid crystal polyester nonwoven fabric or the like and then drying and removing the solvent, or as a curable film which is obtained by applying such solution to a base material such as a polycarbonate film, a polyethylene terephthalate film, an ethylene tetrafluoroethylene copolymer film, a polyimide film, a copper foil, an aluminum foil, a glass plate or a SUS plate.

The thus-obtained curable resin composition is useful for a variety of applications such as an insulating material for a printed wiring board, a resin for a resist, a semiconductor package material, a resin for sealing semiconductor, an adhesive for a printed wiring board, a buildup laminate material, a resin for a fiber-reinforced plastic, a sealing resin for a liquid crystal display panel, a resin for a color filter of a liquid crystal, a coating, a variety of coating agents and an adhesive.

The cured product provided by the present invention can be obtained by curing the curable resin composition of the present invention obtained by the aforementioned process according to a known method such as a curing method which utilizes electron beam, ultraviolet ray or heat. When the curable resin composition is cured by utilizing ultraviolet ray, a low-pressure mercury lamp, a medium-pressure mercury lamp, a high-pressure mercury lamp, an extra-high-pressure mercury lamp, a xenon lamp or a metal halide lamp can be used as a light source of ultraviolet ray. When the curable resin composition is cured by heat, the cured product is obtained by filling the curable resin composition of the present invention into a metallic mold at such a temperature that the resin composition is molten, and then heating the resin composition up to a predetermined polymerization temperature or higher, to allow the resin composition to undergo a cross-linking reaction. The curing temperature is preferably 100 to 300 °C. The curing time is preferably 0.1 to 5 hours.

### Examples

The present invention will be more concretely explained with reference to Examples hereinafter, while the present invention shall not be specially limited to these Examples. A number average molecular weight and a weight average molecular weight were obtained by a gel permeation chromatography (GPC) method (calculated as polystyrene). Tetrahydrofuran (THF) was used for a developing solvent for GPC. A hydroxyl group equivalent and an amino group equivalent were obtained by quantification of a terminal functional group by means of titration. A dielectric constant was measured at 10 GHz by a cavity resonance perturbation method. In Examples of the present invention, the term "at a dried time" means the state of a sample after the sample was kept in a calcium chloride-containing desiccator (humidity 21%) at a temperature of 20 °C for 12 hours and the term "at a moisture-absorbed time" means the state of a sample after the sample was immersed in water having a temperature of 25 °C for 24 hours. A glass transition temperature of a cured product was obtained by DMA method and a glass transition temperature of a cured film was obtained by TMA method. In the DMA method, the glass transition temperature was obtained from an extrapolation point of a storage elastic modulus (E'). In the measurement, a sample having a size of 10 mm x 55mm x about 1 mm was measured for a glass transition temperature under a clamped-clamped beam bending condition at a distance between chucks of 20 mm at a frequency of 10 Hz at an amplitude of 10 µm and at a temperature-increasing rate of 5°C/min. In the TMA method, a film having a size of 3 mm x 30 mm as a sample for measurement was measured for a glass transition temperature at a loading of 5 g at a distance between chucks of 10 mm and at a temperature-increasing rate of 10 °/min by a tensile method. As for solvent solubility, a 20 wt% solution of a sample was prepared at room temperature. "o" represents that no remainder of the sample was confirmed by visual observation. "Δ" represents that partial dissolution of the sample was confirmed by visual observation. "x" represents that the dissolution of the sample was not confirmed by visual observation. As for a water absorption coefficient, a cured product was immersed in hot water having a temperature of 80 °C for 24 hours and then the water absorption coefficient was calculated from a weight change between before and after the immersion.

Synthetic Example 1

### (Synthesis of Bifunctional Phenylene Ether Oligomer)

A 12L longitudinally long reactor equipped with a stirrer, a thermometer, an air-introducing tube and baffleplates was charged with 3.88 g (17.4 mmol) of CuBr₂, 0.75 g (4.4 mmol) of N,N'-di-t-butylethylenediamine, 28.04 g (277.6 mmol) of n-butyldimethylamine and 2,600 g of toluene. The mixture was stirred at a reaction temperature of 40 °C. Separately, 129.32 g (0.48 mol) of 2,2',3,3',5,5'-hexamethyl-(1,1'-biphenyl)-4,4'-diol, 292.19 g (2.40 mol) of 2,6-dimethylphenol, 0.51 g (2.9 mmol) of N,N'-di-t-butylethylenediamine and 10.90 g (108.0 mmol) of n-butyldimethylamine were dissolved in 2,300 g of methanol, to obtain a mixed solution. The mixed solution was dropwise added to the mixture in the reactor over 230 minutes with stirring while bubbling was carried out with a nitrogen-air mixed gas having an oxygen concentration of 8 % at a flow velocity of 5.2L/min. After the completion of the addition, 1,500 g of water containing 19.89 g (52.3 mmol) of tetrasodium ethylenediamine tetraacetate dissolved therein was added to the mixture to terminate the reaction. An aqueous layer and an organic layer were separated. The organic layer was washed with 1N hydrochloric acid aqueous solution and then washed with pure water. The thus-obtained solution was concentrated with an evaporator and then dried under reduced pressure at 120 °C for 3 hours, to obtain 401.5 g of a bifunctional phenylene ether oligomer (resin "A"). The resin "A" had a number average molecular weight of 1, 035, a weight average molecular weight of 1,598 and a hydroxyl group equivalent of 435.

### (Synthesis of Bifunctional Phenylene Ether Oligomer Dinitro Compound)

A 2L reactor vessel equipped with a stirrer, a reflux condenser and a thermometer was charged with 999.3 g of N,N-dimethylacetamide, 250.4 g of the resin "A", 89.4 g (0.63 mol) of 4-fluoronitrobenzene and 95.3 g (0.69 mol) of potassium carbonate. The atmosphere in the reactor vessel was replaced with nitrogen. Then, the resultant mixture was heated and the mixture was continuously stirred at 110°C for 5 hours, to allow the mixture to react. After the completion of the reaction, filtering was carried out at 90 to 100°C to remove an inorganic salt. Then, the thus-obtained filtrate was cooled down to room temperature. The filtrate was poured to a mixed solvent of 1,000 g of methanol and 500 g of pure water, to precipitate a solid. The solid was recovered by filtering, washed with methanol and dried, to obtain 286.9 g of a bifunctional phenylene ether oligomer dinitro compound (resin "B"). The resin "B" had a number average molecular weight of 1,246 and a weight average molecular weight of 1,779. An infrared absorption spectrum (IR) of the resin "B" showed absorptions at a wavenumber of 1, 520cm⁻¹ and a wavenumber of 1,343 cm⁻¹, which correspond to an N-O bond.

### (Synthesis of Bifunctional Phenylene Ether Oligomer Diamine)

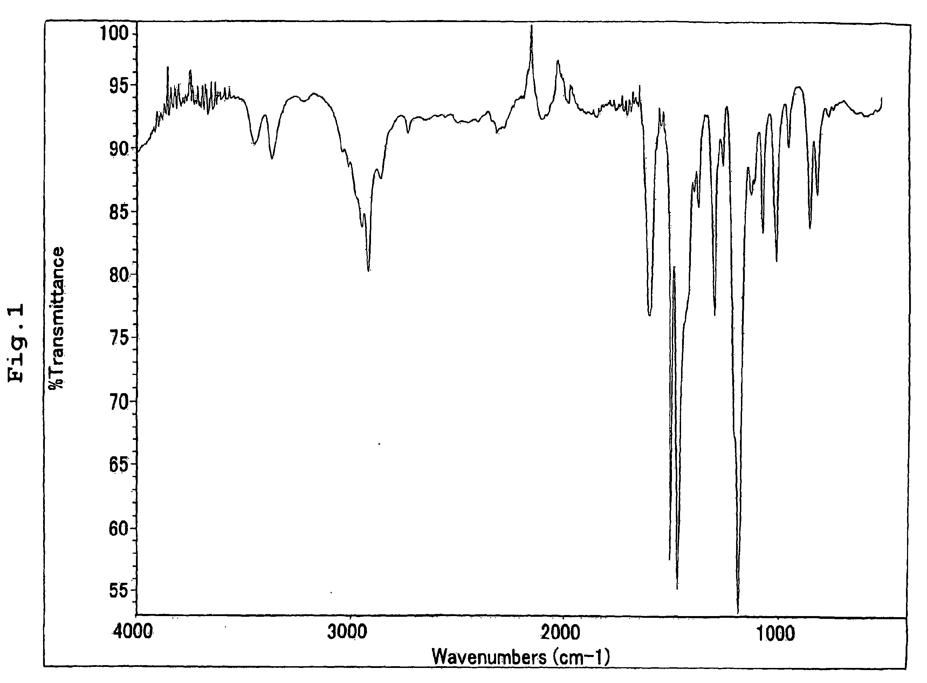

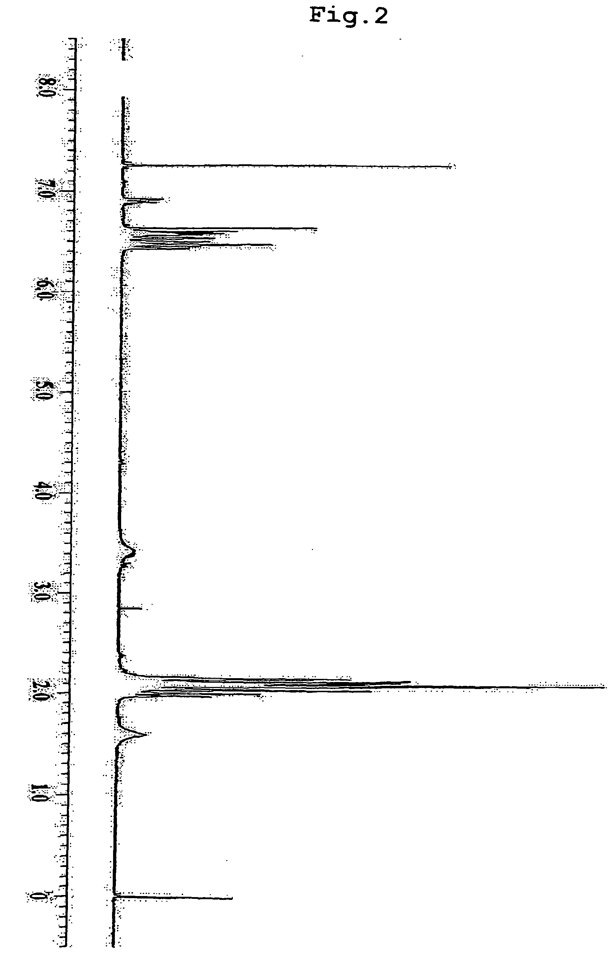

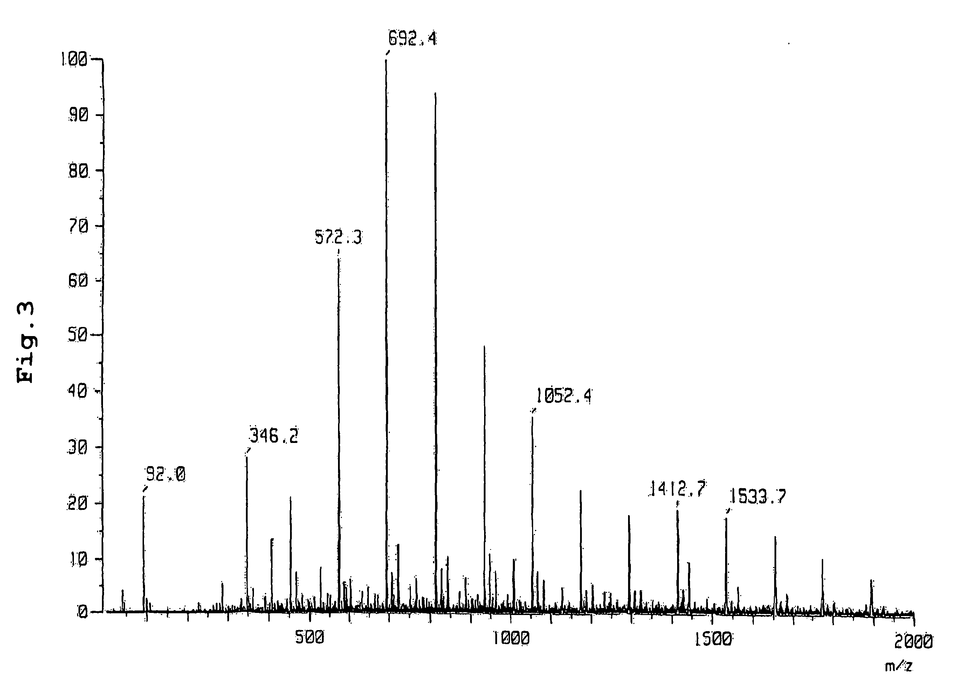

Then, a 2L reactor vessel equipped with a stirrer was charged with 100.0 g of the resin "B", 600 g of N, N-dimethylacetamide and 2.5 g of a 5%Pd/alumina catalyst. The mixture was allowed to react under a hydrogen atmosphere at 80 °C for 7.5 hours with stirring. Then, the resultant reaction solution was filtered to remove the catalyst and then poured to 1,000 g of pure water, to precipitate a solid. The solid was recovered by filtering, washed with pure water and dried, to obtain 85.1 g of a bifunctional phenylene ether oligomer diamine (resin "C"). The resin "C" had a number average molecular weight of 1,269, a weight average molecular weight of 1,788 and an amino group equivalent of 590. As shown in Fig. 1, an infrared absorption spectrum (IR) of the resin "C" showed absorptions at a wavenumber of 3,448 cm⁻¹ and a wavenumber of 3, 367 cm⁻¹, which correspond to an N-H bond. As shown in Fig. 2, a peak of a proton corresponding to an amino group was observed around 3.5 ppm in a ¹H NMR spectrum of the resin "C". An oligomer structure as shown in Fig. 3 was observed in a FD mass spectrum of the resin "C", which agrees with the theoretical molecular weight of the resin "C".

Synthetic Example 2

### (Synthesis of Bifunctional Phenylene Ether Oligomer)

A 12L longitudinally long reactor equipped with a stirrer, a thermometer, an air-introducing tube and baffleplates was charged with 9. 36 g (42.1 mmol) of CuBr₂, 1.81 g (10.5 mmol) of N,N'-di-t-butylethylenediamine, 67.77 g (671.0 mmol) of n-butyldimethylamine and 2,600 g of toluene. The mixture was stirred at a reaction temperature of 40 °C. Separately, 129.32 g (0.48 mol) of 2,2'-,3,3'-,5,5'-hexamethyl-(1,1'-biphenyl)-4,4'-diol, 878.4 g (7.2 mol) of 2,6-dimethylphenol, 1.22 g (7.2 mmol) of N,N'-di-t-butylethylenediamine and 26.35 g (260.9 mmol) of n-butyldimethylamine were dissolved in 2,300 g of methanol, to obtain a mixed solution. The mixed solution was dropwise added to the mixture in the reactor over 230 minutes with stirring while bubbling was carried out with a nitrogen-air mixed gas having an oxygen concentration of 8 % at a flow velocity of 5.2L/min. After the completion of the addition, 1,500 g of water containing 48.06 g (126.4 mmol) of tetrasodium ethylenediamine tetraacetate dissolved therein was added to the mixture to terminate the reaction. An aqueous layer and an organic layer were separated. Then, the organic layer was washed with 1N hydrochloric acid aqueous solution and then washed with pure water. The thus-obtained solution was concentrated with an evaporator and then dried in vacuum at 120 °C for 3 hours, to obtain 844 g of a bifunctional phenylene ether oligomer (resin "D"). The resin "D" had a number average molecular weight of 1,975, a weight average molecular weight of 3,514 and a hydroxyl group equivalent of 990.

### (Synthesis of Bifunctional Phenylene Ether Oligomer Dinitro Compound)

A 500ml reactor vessel equipped with a stirrer, a reflux condenser, a thermometer and a Dean and Stark water separator was charged with 250.2 g of N,N-dimethylformamide, 148.5 g of the resin "D", 52.1 g (0.33 mol) of 4-chloronitrobenzene and 25.0 g (0.18 mol) of potassium carbonate. 20.0 g of toluene was added to the reactor vessel. The atmosphere in the reactor vessel was replaced with nitrogen. Then, the resultant mixture was heated and the mixture was continuously stirred for 5 hours at a temperature of 140 to 150°C, to allow the mixture to react. Water generated by the reaction was sequentially removed by azeotropy with toluene. After the completion of the reaction, filtering was carried out at 80 to 90°C, to remove an inorganic salt. Then, the thus-obtained filtrate was cooled down to room temperature. The filtrate was poured to 320.1 g of methanol, to precipitate a solid. The solid was recovered by filtering, washed with methanol and then dried, to obtain 140.3 g of a bifunctional phenylene ether oligomer dinitro compound (resin "E"). The resin "E" had a number average molecular weight of 3,081 and a weight average molecular weight of 5,587. An infrared absorption spectrum (IR) of the resin "E" showed absorptions at a wavenumber of 1, 519 cm⁻¹ and a wavenumber of 1,342 cm⁻¹, which correspond to an N-O bond.

### (Synthesis of Bifunctional Phenylene Ether Oligomer Diamine)

Then, a reactor vessel equipped with a stirrer was charged with 36.00 g of the resin "E", 1050 g of N,N-dimethylacetamide and 4.68 g of a 5%Pd/alumina catalyst. The mixture was allowed to react under a hydrogen atmosphere at 80 °C for 8 hours with stirring. Then, the resultant reaction solution was filtered to remove the catalyst and then pored into 1,050 g of pure water, to precipitate a solid. The solid was recovered by filtering, washed with pure water and dried, to obtain 29.7 g of a bifunctional phenylene ether oligomer diamine (resin "F"). The resin "F" had a number average molecular weight of 2,905, a weight average molecular weight of 6,388 and an amino group equivalent of 1,351. An infrared absorption spectrum (IR) of the resin "F" showed absorptions at a wavenumber of 3,447 cm⁻¹ and a wavenumber of 3,365 cm⁻¹, which correspond to an N-H bond.

### Synthetic Example 3

### (Synthesis of Bifunctional Phenylene Ether Oligomer)

A 12L longitudinally long reactor equipped with a stirrer, a thermometer, an air-introducing tube and baffleplates was charged with 13.1 g (0.12 mol) of CuCl, 707.0 g (5.5 mol) of di-n-butylamine and 4,000 g of methyl ethyl ketone. The mixture was stirred at a reaction temperature of 40 °C. Separately, 410.2 g (1.6 mol) of 4,4'-methylenebis(2,6-dimethylphenol) and 586.5 g (4.8 mol) of 2,6-dimethylphenol were dissolved in 8,000 g of methyl ethyl ketone to obtain a solution. The solution was dropwise added to the mixture in the reactor over 120 minutes while bubbling was carried out with 2 L/min of air. A disodium dihydrogen ethylenediamine tetraacetate aqueous solution was added to the resultant mixture to terminate the reaction. Then, washing was carried out with 1N hydrochloric acid aqueous solution three times and then washing was carried out with ion-exchanged water. The thus-obtained solution was concentrated with an evaporator and then dried under reduced pressure, to obtain 946.6 g of a bifunctional phenylene ether oligomer (resin "G"). The resin "G" had a number average molecular weight of 801, a weight average molecular weight of 1,081 and a hydroxyl group equivalent of 455.

### (Synthesis of Bifunctional Phenylene Ether Oligomer Dinitro Compound)

A 500ml reactor vessel equipped with a stirrer, a reflux condenser, a thermometer and a Dean and Stark water separator was charged with 200.2 g of N,N-dimethylformamide, 68.3 g of the resin "G", 52.2 g (0.33 mol) of 4-chloronitrobenzene and 24.9 g (0.18 mol) of potassium carbonate. 19.0 g of toluene was added to the reactor vessel. The atmosphere in the reactor vessel was replaced with nitrogen. Then, the resultant mixture was heated and the mixture was continuously stirred for 5 hours at a temperature of 140 to 150°C, to allow the mixture to react. Water generated by the reaction was sequentially removed by azeotropy with toluene. After the completion of the reaction, filtering was carried out at 80 to 90°C, to remove an inorganic salt. Then, the thus-obtained filtrate was cooled down to room temperature. The filtrate was poured to 290.2 g of methanol, to precipitate a solid. The solid was recovered by filtering, washed with methanol and then dried, to obtain 63.8 g of a bifunctional phenylene ether oligomer dinitro compound (resin "H"). The resin "H" had a number average molecular weight of 1, 250 and a weight average molecular weight of 1,719. An infrared absorption spectrum (IR) of the resin "H" showed absorptions at a wavenumber of 1, 522 cm⁻¹ and a wavenumber of 1,340 cm⁻¹, which correspond to an N-O bond.

### (Synthesis of Bifunctional Phenylene Ether Oligomer Diamine)

Then, a reactor vessel equipped with a stirrer was charged with 34.5 g of the resin "H", 900 g of N,N-dimethylacetamide and 4.8 g of a 5%Pd/alumina catalyst. The mixture was allowed to react under a hydrogen atmosphere at 80°C for 7 hours with stirring. Then, the resultant reaction solution was filtered to remove the catalyst and then pored into 900 g of pure water, to precipitate a solid. The solid was recovered by filtering, washed with pure water and dried, to obtain 26.4 g of a bifunctional phenylene ether oligomer diamine (resin "I"). The resin "I" had a number average molecular weight of 1,205, a weight average molecular weight of 2,009 and an amino group equivalent of 560. An infrared absorption spectrum (IR) of the resin "I" showed absorptions at a wavenumber of 3,446 cm⁻¹ and a wavenumber of 3,367 cm⁻¹, which correspond to an N-H bond.

Synthetic Example 4

### (Synthesis of Bifunctional Phenylene Ether Oligomer)

A 12L longitudinally long reactor equipped with a stirrer, a thermometer, an air-introducing tube and baffleplates was charged with 13.1 g (0.12 mol) of CuCl, 707.0 g (5.5 mol) of di-n-butylamine and 4,000 g of methyl ethyl ketone. The mixture was stirred at a reaction temperature of 40 °C. Separately, 82.1 g (0.32 mol) of 4,4'-methylenebis(2,6-dimethylphenol) and 586.5 g (4.8 mol) of 2,6-dimethylphenol were dissolved in 8,000 g of methyl ethyl ketone to obtain a solution. The solution was dropwise added to the mixture in the reactor over 120 minutes while bubbling was carried out with 2 L/min of air. A disodium dihydrogen ethylenediamine tetraacetate aqueous solution was added to the resultant mixture, to terminate the reaction. Then, washing was carried out with 1N hydrochloric acid aqueous solution three times and then washing was carried out with ion-exchanged water. The thus-obtained solution was concentrated with an evaporator and then dried under reduced pressure, to obtain 632.5 g of a bifunctional phenylene ether oligomer (resin "J"). The resin "J" had a number average molecular weight of 1, 884, a weight average molecular weight of 3,763 and a hydroxyl group equivalent of 840.

### (Synthesis of Bifunctional Phenylene Ether Oligomer Dinitro Compound)

A 500ml reactor vessel equipped with a stirrer, a reflux condenser, a thermometer and a Dean and Stark water separator was charged with 250.5 g of N,N-dimethylformamide, 126.0 g of the resin "J", 51.9 g (0.33 mol) of 4-chloronitrobenzene and 25.0 g (0. 18 mol) of potassium carbonate. 19.2 g of toluene was added to the reactor vessel. The atmosphere in the reactor vessel was replaced with nitrogen. Then, the resultant mixture was heated and the mixture was continuously stirred for 5 hours at a temperature of 140 to 150°C, to allow the mixture to react. Water generated by the reaction was sequentially removed by azeotropy with toluene. After the completion of the reaction, filtering was carried out at 80 to 90°C, to remove an inorganic salt. Then, the thus-obtained filtrate was cooled down to room temperature. The filtrate was poured to 330.3 g of methanol, to precipitate a solid. The solid was recovered by filtering, washed with methanol and then dried, to obtain 115.0 g of a bifunctional phenylene ether oligomer dinitro compound (resin "K"). The resin "K" had a number average molecular weight of 2, 939 and a weight average molecular weight of 5,982. An infrared absorption spectrum (IR) of the resin "K" showed absorptions at a wavenumber of 1, 518 cm⁻¹ and a wavenumber of 1,343 cm⁻¹, which correspond to an N-O bond.

### (Synthesis of Bifunctional Phenylene Ether Oligomer Diamine)

Then, a reactor vessel equipped with a stirrer was charged with 42.6 g of the resin "K", 700 g of N,N-dimethylacetamide and 1.89 g of a 5%Pd/alumina catalyst. The mixture was allowed to react under a hydrogen atmosphere at 80 °C for 8 hours with stirring. Then, the resultant reaction solution was filtered to remove the catalyst and then pored into 1,000 g of pure water, to precipitate a solid. The solid was recovered by filtering, washed with pure water and dried, to obtain 32.3 g of a bifunctional phenylene ether oligomer diamine (resin "L"). The resin "L" had a number average molecular weight of 2,733, a weight average molecular weight of 6,746 and an amino group equivalent of 1,271. An infrared absorption spectrum (IR) of the resin "L" showed absorptions at a wavenumber of 3,449 cm⁻¹ and a wavenumber of 3,366 cm⁻¹, which correspond to an N-H bond.

Synthetic Example 5

### (Synthesis of Bifunctional Phenylene Ether Oligomer)

A 2L longitudinally long reactor equipped with a stirrer, a thermometer, an air-introducing tube and baffleplates was charged with 18.0 g (78.8 mmol) of 2, 2-bis (4-hydroxyphenyl) propane (bisphenol A), 0.172 g (0.77 mmol) of CuBr₂, 0.199 g (1.15 mmol) of N,N'-di-t-butylethylenediamine, 2.10 g (2.07 mmol) of n-butyldimethylamine, 139 g of methanol and 279 g of toluene. Separately, 48.17 g (0.394 mol) of 2,6-dimethylphenol, 0.245 g (1.44 mmol) of N,N'-di-t-butylethylenediamine and 2.628 g (25.9 mmol) of n-butyldimethylamine were dissolved in 133 g of methanol and 266 g of toluene, to obtain a mixed solution. The mixed solution was dropwise added to the reactor, in which the mixture was stirred at a liquid temperature of 40°C, over 132 minutes while bubbling was carried with air at a flow velocity of 0.5 L/min. After the completion of the addition of the mixed solution, the resultant mixture was further stirred for 120 minutes. 400 g of water containing 2.40 g of tetrasodium ethylenediamine tetraacetate dissolved therein was added to the stirred mixture to terminate the reaction. An aqueous layer and an organic layer were separated. Then, washing with pure water was carried out. The thus-obtained solution was concentrated with an evaporator and then dried in vacuum at 120°C for 3 hours, to obtain 54.8 g of a bifunctional phenylene ether oligomer (resin "M") . The resin "M" had a number average molecular weight of 1,348, a weight average molecular weight of 3,267 and a hydroxyl group equivalent of 503.

### (Synthesis of Bifunctional Phenylene Ether Oligomer Dinitro Compound)

A 300ml reactor vessel equipped with a stirrer, a reflux condenser, a thermometer and a Dean and Stark water separator was charged with 100.1 g of N,N-dimethylformamide, 37.7 g of the resin "M", 26.0 g (0.16 mol) of 4-chloronitrobenzene and 12.5 g (0.09 mol) of potassium carbonate. 10.0 g of toluene was added to the reactor vessel. The atmosphere in the reactor vessel was replaced with nitrogen. Then, the resultant mixture was heated and the mixture was continuously stirred for 5 hours at a temperature of 140 to 150°C, to allow the mixture to react. Water generated by the reaction was sequentially removed by azeotropy with toluene. After the completion of the reaction, filtering was carried out at 80 to 90°C, to remove an inorganic salt. Then, the thus-obtained filtrate was cooled down to room temperature. The filtrate was poured to 150.1 g of methanol, to precipitate a solid. The solid was recovered by filtering, washed with methanol and then dried, to obtain 36.0 g of a bifunctional phenylene ether oligomer dinitro compound (resin "N"). The resin "N" had a number average molecular weight of 2,103 and a weight average molecular weight of 5,194. An infrared absorption spectrum (IR) of the resin "N" showed absorptions at a wavenumber of 1, 516 cm⁻¹ and a wavenumber of 1,340 cm⁻¹, which correspond to an N-O bond.

### (Synthesis of Bifunctional Phenylene Ether Oligomer Diamine)

Then, a reactor vessel equipped with a stirrer was charged with 26.2 g of the resin "N", 600 g of N,N-dimethylacetamide and 3.30 g of a 5%Pd/alumina catalyst. The mixture was allowed to react under a hydrogen atmosphere at 80 °C for 9 hours with stirring. Then, the resultant reaction solution was filtered to remove the catalyst and then pored into 600 g of pure water, to precipitate a solid. The solid was recovered by filtering, washed with pure water and dried, to obtain 22.0 g of a bifunctional phenylene ether oligomer diamine (resin "O") . The resin "O" had a number average molecular weight of 2,051, a weight average molecular weight of 6, 42 and an amino group equivalent of 954. An infrared absorption spectrum (IR) of the resin "O" showed absorptions at a wavenumber of 3, 450 cm⁻¹ and a wavenumber of 3,365 cm⁻¹, which correspond to an N-H bond.

Synthetic Example 6

### (Synthesis of Bifunctional Phenylene Ether Oligomer)

A 12L longitudinally long reactor equipped with a stirrer, a thermometer, an air-introducing tube and baffleplates was charged with 3.88 g (17.4 mmol) of CuBr₂, 0.75 g (4.4 mmol) of N,N'-di-t-butylethylenediamine, 28.04 g (277.6 mmol) of n-butyldimethylamine and 2,600 g of toluene. The mixture was stirred at a reaction temperature of 40°C. Separately, 129.3 g (0.48 mol) of 2,2'-,3,3'-,5,5'-hexamethyl-(1,1'-biphenyl)-4,4'-diol, 233.7 g (1.92 mol) of 2,6-dimethylphenol, 64.9 g (0.48 mol) of 2,3,6-trimethylphenol, 0.51 g (2.9 mmol) of N,N'-di-t-butylethylenediamine and 10.90 g (108.0 mmol) of n-butyldimethylamine were dissolved in 2, 300 g of methanol, to obtain a mixed solution. The mixed solution was dropwise added to the mixture in the reactor over 230 minutes with stirring while bubbling was carried out with a nitrogen-air mixed gas having an oxygen concentration of 8 % at a flow velocity of 5.2L/min. After the completion of the addition, 1,500 g of water containing 19.89 g (52.3 mmol) of tetrasodium ethylenediamine tetraacetate dissolved therein was added to the mixture to terminate the reaction. An aqueous layer and an organic layer were separated. The organic layer was washed with 1N hydrochloric acid aqueous solution and then washed with pure water. The thus-obtained solution was concentrated with an evaporator and then dried in vacuum at 120°C for 3 hours, to obtain 410.2 g of a bifunctional phenylene ether oligomer (resin "P"). The resin "P" had a number average molecular weight of 986, a weight average molecular weight of 1,530 and a hydroxyl group equivalent of 471.

### (Synthesis of Bifunctional Phenylene Ether Oligomer Dinitro Compound)

A 500ml reactor vessel equipped with a stirrer, a reflux condenser, a thermometer and a Dean and Stark water separator was charged with 200.0 g of N,N-dimethylformamide, 70.7 g of the resin "P", 52.0 g (0.33 mol) of 4-chloronitrobenzene and 25.1 g (0. 18 mol) of potassium carbonate. 19.3 g of toluene was added to the reactor vessel. The atmosphere in the reactor vessel was replaced with nitrogen. Then, the resultant mixture was heated and the mixture was continuously stirred for 5 hours at a temperature of 140 to 150°C, to allow the mixture to react. Water generated by the reaction was sequentially removed by azeotropy with toluene. After the completion of the reaction, filtering was carried out at 80 to 90°C, to remove an inorganic salt. Then, the thus-obtained filtrate was cooled down to room temperature. The filtrate was poured to 300.3 g of methanol, to precipitate a solid. The solid was recovered by filtration, washed with methanol and then dried, to obtain 64.1 g of a bifunctional phenylene ether oligomer dinitro compound (resin "Q"). The resin "Q" had a number average molecular weight of 1, 538 and a weight average molecular weight of 2,432. An infrared absorption spectrum (IR) of the resin "Q" showed absorptions at a wavenumber of 1, 522 cm⁻¹ and a wavenumber of 1,344 cm⁻¹, which correspond to an N-O bond.

### (Synthesis of Bifunctional Phenylene Ether Oligomer Diamine)

Then, a reactor vessel equipped with a stirrer was charged with 30.0 g of the resin "Q", 600 g of N,N-dimethylacetmamide and 3.4 g of a 5%Pd/alumina catalyst. The mixture was allowed to react under a hydrogen atmosphere at 80 °C for 7.5 hours with stirring. Then, the resultant reaction solution was filtered to remove the catalyst and then pored into 600 g of pure water, to precipitate a solid. The solid was recovered by filtering, washed with pure water and dried, to obtain 22.8 g of a bifunctional phenylene ether oligomer diamine (resin "R"). The resin "R" had a number average molecular weight of 1,465, a weight average molecular weight of 2,809 and an amino group equivalent of 681. An infrared absorption spectrum (IR) of the resin "R" showed absorptions at a wavenumber of 3,447 cm⁻¹ and a wavenumber of 3,360 cm⁻¹, which correspond to an N-H bond.

### Example 1

### (Synthesis of Bismaleamic Acid)

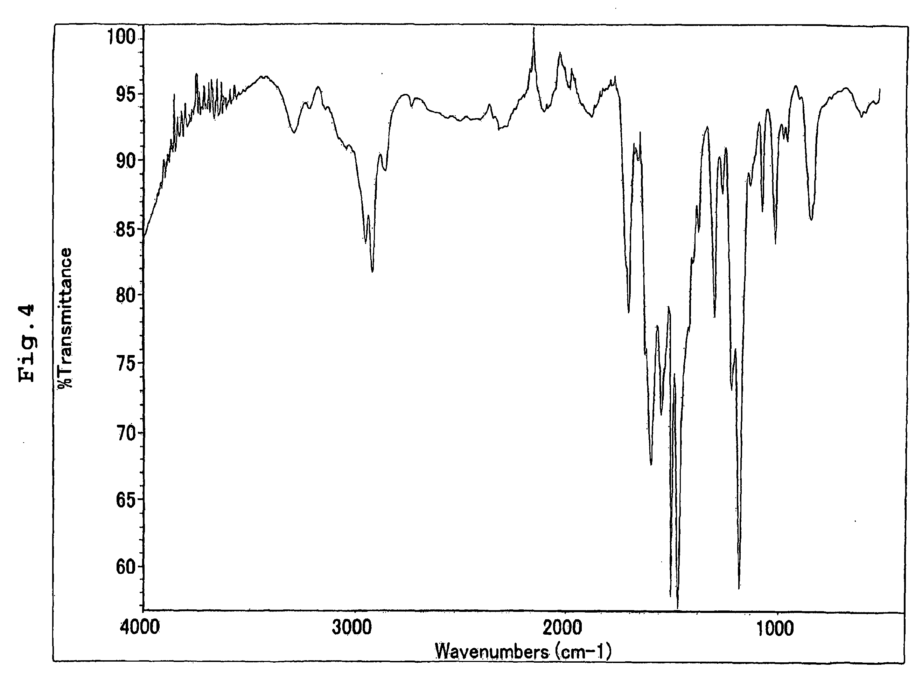

A nitrogen gas was introduced into a container equipped with a stirrer, a reflux condenser and a nitrogen-introducing tube. 11.80 g of the resin "C" was charged into the container. Then, 96.43 g of ethyl acetate was added and the resin "C" was fully dissolved therein. Then, 1.987 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 13.73 g of a bismaleamic acid (resin "S"). In an IR spectrum of the resin "S", as shown in Fig.4, a N-H stretching vibration of an amide group was observed at 3,294 cm⁻¹ and a stretching vibration of a C-O double bond of a carboxyl group and a stretching vibration of a C-O double bond of an amide group were observed at 1,710 cm⁻¹.

### Example 2

### (Synthesis of Bismaleamic Acid)

A nitrogen gas was introduced into a container equipped with a stirrer, a reflux condenser and a nitrogen-introducing tube. 27.02 g of the resin "F" was charged into the container. Then, 202.87 g of ethyl acetate was added and the resin "F" was fully dissolved therein. Then, 1. 961 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 28.40 g of a bismaleamic acid (resin "T").

### Example 3

### (Synthesis of Bismaleamic Acid)

A nitrogen gas was introduced into a container equipped with a stirrer, a reflux condenser and a nitrogen-introducing tube. 11.20 g of the resin "I" was charged into the container. Then, 91.72 g of ethyl acetate was added and the resin "I" was fully dissolved therein. Then, 1.963 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 12.77 g of a bismaleamic acid (resin "U").

### Example 4

### (Synthesis of Bismaleamic Acid)

A nitrogen gas was introduced into a container equipped with a stirrer, a reflux condenser and a nitrogen-introducing tube. 25.42 g of the resin "L" was charged into the container. Then, 192.03 g of ethyl acetate was added and the resin "L" was fully dissolved therein. Then, 1.962 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 26.01 g of a bismaleamic acid (resin "V").

### Example 5

### (Synthesis of Bismaleamic Acid)

A nitrogen gas was introduced into a container equipped with a stirrer, a reflux condenser and a nitrogen-introducing tube. 19.08 g of the resin "O" was charged into the container. Then, 147.22 g of ethyl acetate was added and the resin "O" was fully dissolved therein. Then, 1.970 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 20.83 g of a bismaleamic acid (resin "W").

### Example 6

### (Synthesis of Bismaleamic Acid)

A nitrogen gas was introduced into a container equipped with a stirrer, a reflux condenser and a nitrogen-introducing tube. 13.62 g of the resin "R" was charged into the container. Then, 109.17 g of ethyl acetate was added and the resin "R" was fully dissolved therein. Then, 1.962 g of maleic anhydride was added and then the mixture was stirred at 30 °C for 3 hours. The resultant reaction mixture was poured into 500 g of pure water, to precipitate a solid. The solid was recovered by filtering, washed with hexane and dried, to obtain 14.96 g of a bismaleamic acid (resin "X").

### Example 7

### (Synthesis of Bismaleimide)

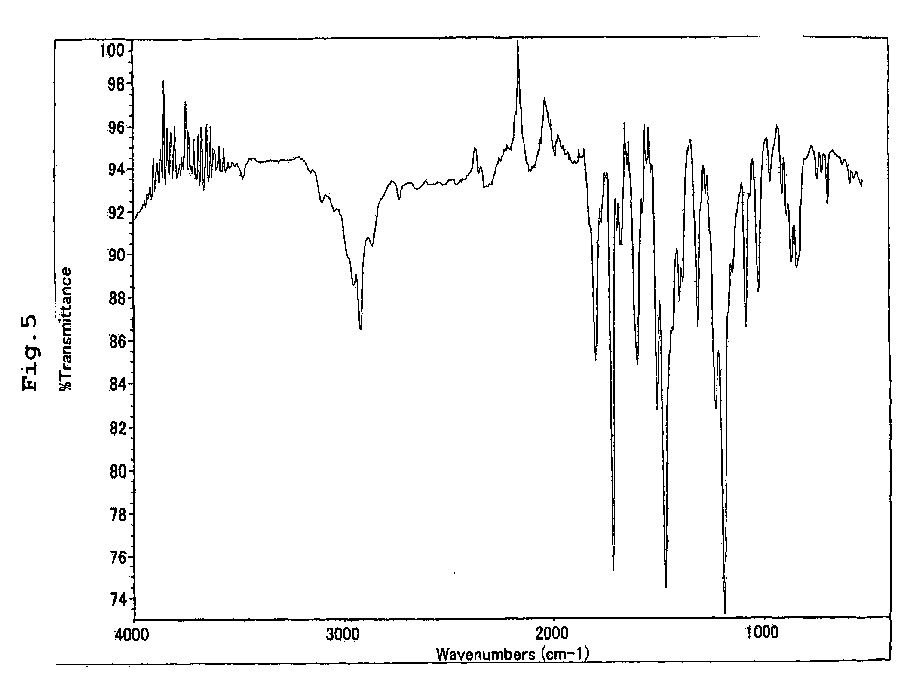

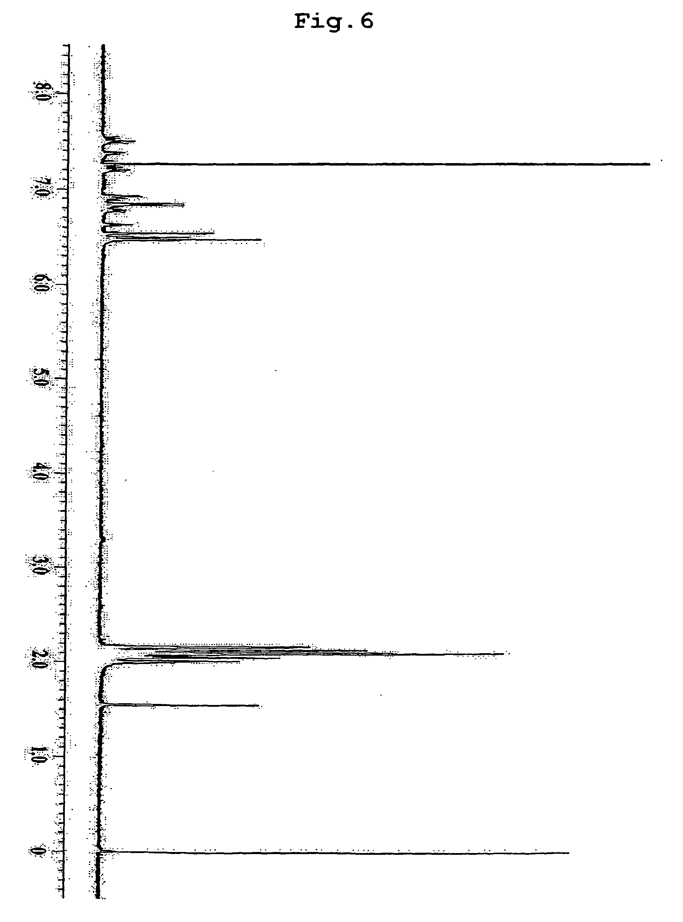

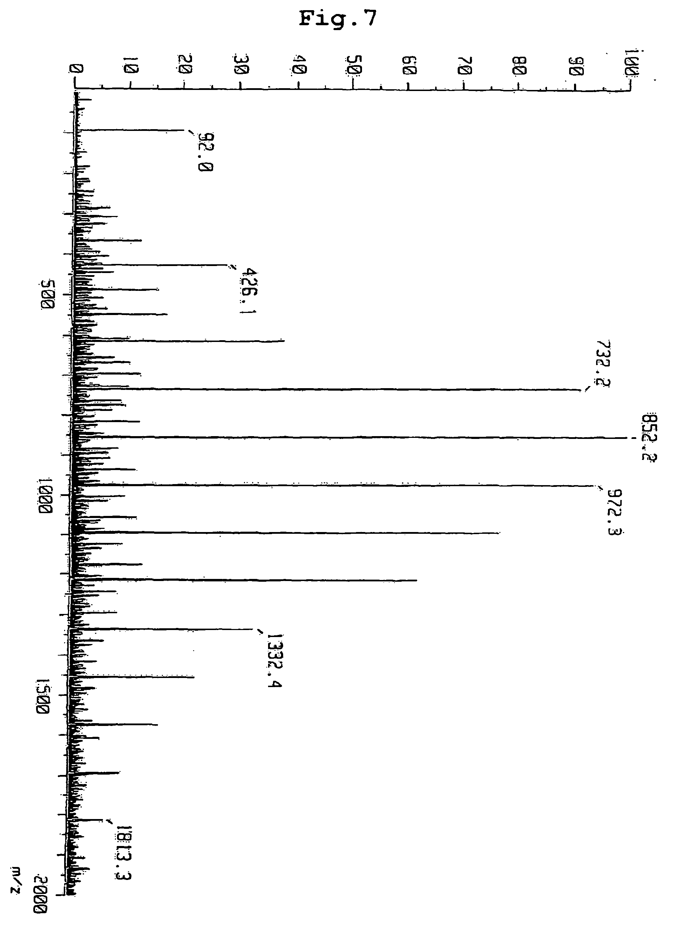

A container containing stir bars was charged with 12.45 g of the bismaleamic acid "S", 181.60 g of acetic anhydride, 40 g of tetrahydrofuran and 7.14 g of potassium acetate. Nitrogen replacement was carried out, and then the mixture was stirred at room temperature for 65 hours. The resultant reaction solution was filtered to remove excess potassium acetate. Then, the resultant solution was poured into 1, 500 g of a pure water : methanol solvent having a weight ratio of 2 : 1, to precipitate a solid. The solid was recovered by filtering, washed with a pure water : methanol solvent having a weight ratio of 2 : 1 and dried, whereby 10.90 g of a bismaleimide "Y" was obtained. In an IR spectrum of the resin "Y", as shown in Fig.5, the N-H stretching vibration of an amide group at 3,294 cm⁻¹ observed in Fig.4 disappeared and a stretching vibration of a C-O double bond of an amide group was observed at 1,710 cm⁻¹. In a ¹H NMR spectrum, as shown in Fig.6, a peak of a proton bonding to a C-C double bond of bismaleimide was observed at 7.0-7.5 ppm. In a FD mass spectrum, as shown in Fig.7, a peak of an oligomer was observed, which agrees with the theoretical molecular weight of a case where an amino group of the resin "C" was converted into a bismaleimide group.

### Example 8

### (Synthesis of Bismaleimide)

A container containing stir bars was charged with 26.08 g of the bismaleamic acid "T", 183.76 g of acetic anhydride, 40 g of tetrahydrofuran and 7.16 g of potassium acetate. Nitrogen replacement was carried out, and then the mixture was stirred at room temperature for 50 hours. The resultant reaction solution was filtered to remove excess potassium acetate. Then, the resultant solution was poured into 2, 000 g of a pure water : methanol solvent having a weight ratio of 2 : 1, to precipitate a solid. The solid was recovered by filtering, washed with a pure water : methanol solvent having a weight ratio of 2 : 1 and dried, whereby 23.22 g of a bismaleimide "Z" was obtained.

### Example 9

### (Synthesis of Bismaleimide)

A container containing stir bars was charged with 11.84 g of the bismaleamic acid "U", 185.29 g of acetic anhydride, 42 g of tetrahydrofuran and 7.10 g of potassium acetate. Nitrogen replacement was carried out, and then the mixture was stirred at room temperature for 65 hours. The resultant reaction solution was filtered to remove excess potassium acetate. Then, the resultant solution was poured into 1, 500 g of a pure water : methanol solvent having a weight ratio of 2 : 1, to precipitate a solid. The solid was recovered by filtering, washed with a pure water : methanol solvent having a weight ratio of 2 : 1 and dried, whereby 10.60 g of a bismaleimide "AA" was obtained.

### Example 10

### (Synthesis of Bismaleimide)

A container containing stir bars was charged with 24.64 g of the bismaleamic acid "V", 180.70 g of acetic anhydride, 42 g of tetrahydrofuran and 6.97 g of potassium acetate. Nitrogen replacement was carried out, and then the mixture was stirred at room temperature for 50 hours. The resultant reaction solution was filtered to remove excess potassium acetate. Then, the resultant solution was poured into 2,000 g of a pure water : methanol solvent having a weight ratio of 2 : 1, to precipitate a solid. The solid was recovered by filtering, washed with a pure water : methanol solvent having a weight ratio of 2 : 1 and dried, whereby 21.55 g of a bismaleimide "AB" was obtained.

### Example 11

### (Synthesis of Bismaleimide)

A container containing stir bars was charged with 18.94 g of the bismaleamic acid "W", 178.15 g of acetic anhydride, 43 g of tetrahydrofuran and 7.36 g of potassium acetate. Nitrogen replacement was carried out, and then the mixture was stirred at room temperature for 60 hours. The resultant reaction solution was filtered to remove excess potassium acetate. Then, the resultant solution was poured into 1, 500 g of a pure water : methanol solvent having a weight ratio of 2 : 1, to precipitate a solid. The solid was recovered by filtering, washed with a pure water : methanol solvent having a weight ratio of 2 : 1 and dried, whereby 15.32 g of a bismaleimide "AC" was obtained.

### Example 12

### (Synthesis of Bismaleimide)

A container containing stir bars was charged with 14.02 g of the bismaleamic acid "X", 183.35 g of acetic anhydride, 43 g of tetrahydrofuran and 7.29 g of potassium acetate. Nitrogen replacement was carried out, and then the mixture was stirred at room temperature for 70 hours. The resultant reaction solution was filtered to remove excess potassium acetate. Then, the resultant solution was poured into 1, 500 g of a pure water : methanol solvent having a weight ratio of 2 : 1, to precipitate a solid. The solid was recovered by filtering, washed with a pure water : methanol solvent having a weight ratio of 2 : 1 and dried, whereby 12.66 g of a bismaleimide "AD" was obtained.

The bismaleimides "Y", "Z", "AA", "AB", "AC" and "AD" obtained in Examples 7 - 12, 2,2-(bis(4-maleimidophenoxy)phenyl)propane (BBMI) and bis(4-maleimidophenyl)methane (BMI) were evaluated with regard to solvent solubility at room temperature. Table 1 shows the results thereof.

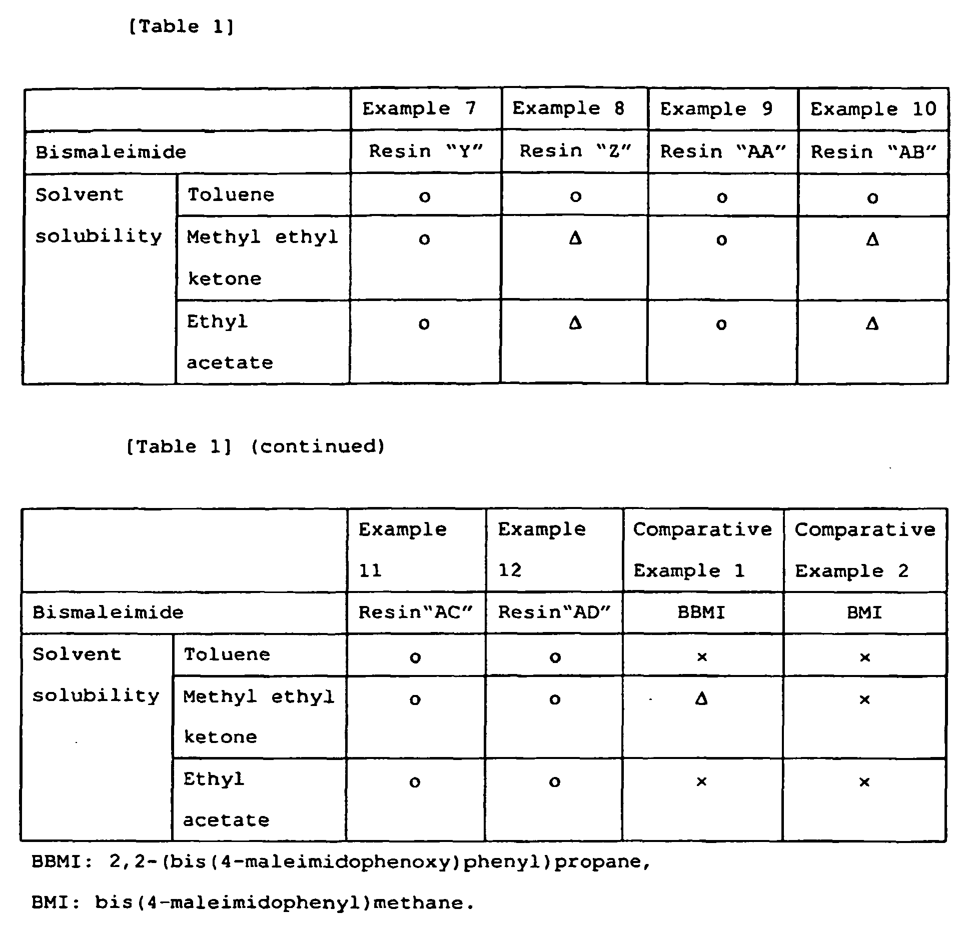

From Table 1, it is found that the bismaleimides of the present invention are excellent in the solubility into general-purpose solvents such as toluene, methyl ethyl ketone and ethyl acetate.

### Examples 13 - 18 and Comparative Examples 3 - 4

The bismaleimide "Y" "Z", "AA", "AB" "AC" or "AD" obtained in Examples 7 - 12, 2,2-(bis(4-maleimidophenoxy)phenyl)propane (BBMI) (BMI-80, supplied by KI Chemical industry Co., Ltd.) or bis (4-maleimidophenyl) methane (BMI) (BMI-1000, supplied by Daiwa Fine Chemicals Co., Ltd.) was pressed with a vacuum press machine by use of a metallic mold made of SUS by heating it up to 250 °C at a temperature-increasing rate of 3 °C/min under 2 MPa and then maintaining it at 250 °C for 3 hours under 2MPa, whereby a resin cured product having a thickness of about 1 mm was obtained. The resin cured product was evaluated with regard to a glass transition temperature (DMA method), dielectric characteristics at a dried time and at a moisture-absorbed time, and a water absorption coefficient. Table 2 shows the results thereof.

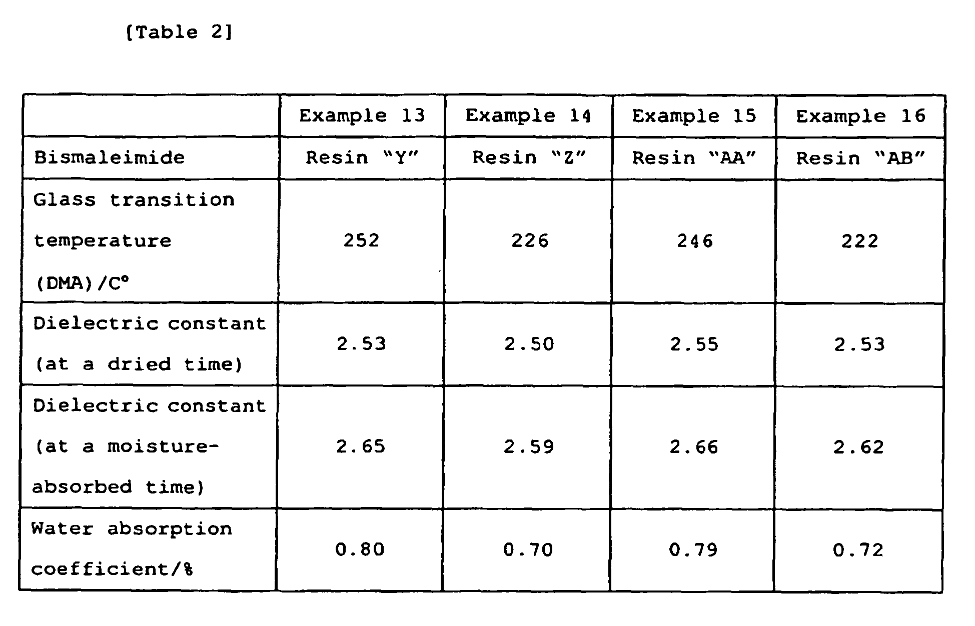

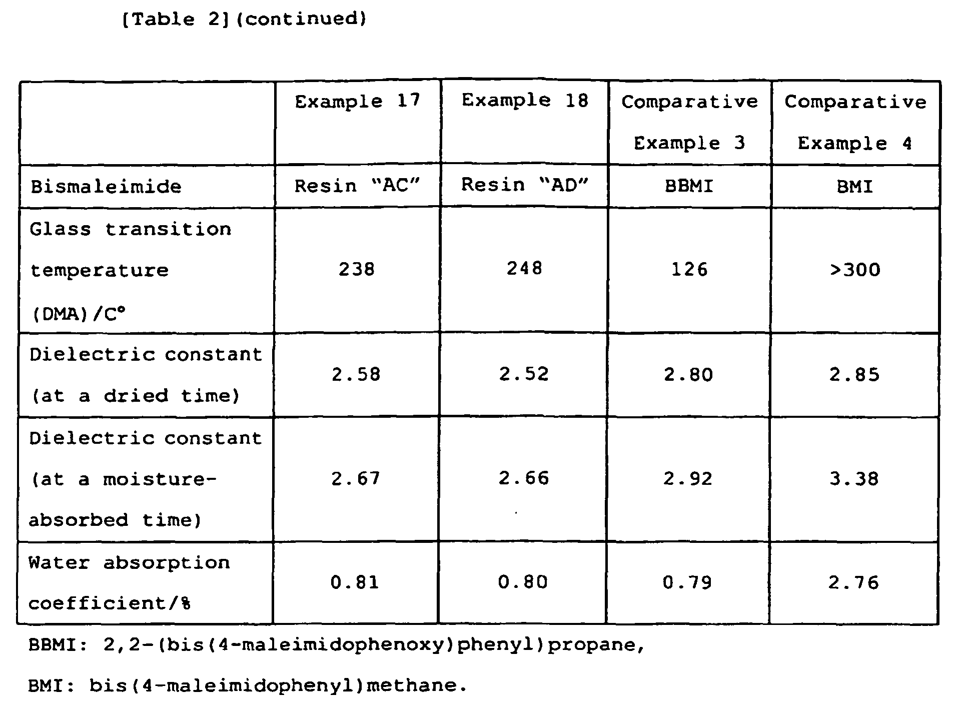

From Table 2, it is found that the cured products obtained by curing the bismaleimides of the present invention have excellent heat resistance and remarkably low dielectric constants, can keep low dielectric characteristics even at a moisture-absorbed time and also have low water absorption coefficients.

### Example 19

5 g of the bismaleimide "Y" obtained in Example 7 and 0.25 g of 2,5-dimethyl-2,5-di(t-butylperoxy)hexane (PERHEXA 25 B, supplied by NOF CORPORATION) were dissolved in 5 g of toluene to prepare a resin composition "AE". The resin composition "AE" was applied to a shiny surface of an 18µm electrolytic copper foil (3EC-III, supplied by Mitsui Mining & Smelting Co., Ltd.) with a doctor blade (gap 400 µm). The applied resin composition "AE" was air-dried at room temperature for 10 minutes and then dried with an air-blowing dryer at 80 °C for 5 minutes, to obtain a base-material-attached curable resin composition having a resin layer with a thickness of about 70 µm. Then, the base-material-attached curable resin composition was heated up to 200 °C with an inert oven under nitrogen at a temperature-increasing rate of 10 °C/min and then maintained at 200 °C for 2 hours. Then, the base material copper foil was removed by etching, to obtain a film. The thickness of the film was about 70 µm. The obtained resin cured film was evaluated with regard to a glass transition temperature (TMA method) and electric characteristics at a dried time and at a moisture-absorbed time. Table 3 shows the results thereof.

**[Table 3]**

| | Example 19 |
|---|---|
| Glass transition temperature | 215 |
| (TMA)/C° | |
| Dielectric constant | 2.54 |
| (at a dried time) | |
| Dielectric constant | 2.58 |
| (at a moisture-absorbed time) | |

## Claims

1. A bismaleimide represented by the formula (1), 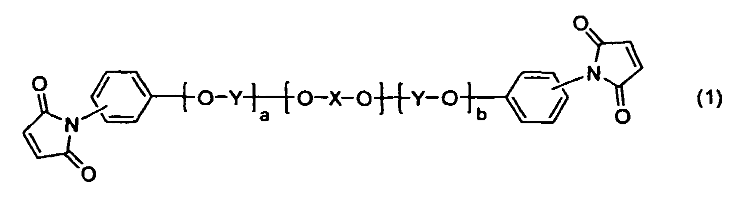 wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each imide group is a para-position or a meta-position, 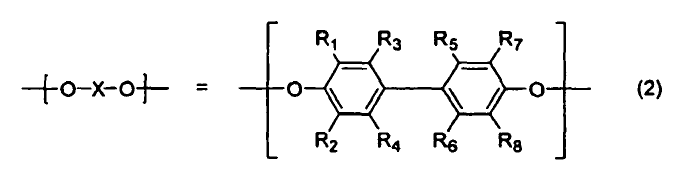 wherein R₁, R₂, R₃, R₇ and R₈ are the same or different and represent a halogen atom, an alkyl group having 6 or less carbon atoms or a phenyl group, R₄, R₅ and R₆ are the same or different and represent a hydrogen atom, a halogen atom, an alkyl group having 6 or less carbon atoms or a phenyl group, 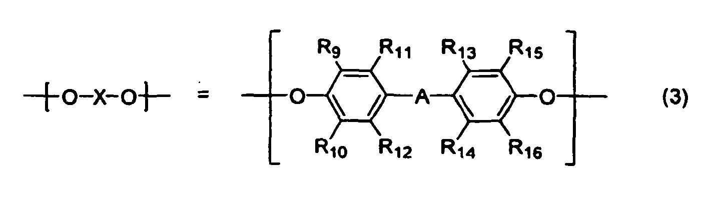 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- is a linear, branched or cyclic bivalent hydrocarbon group having 20 or less carbon atoms, 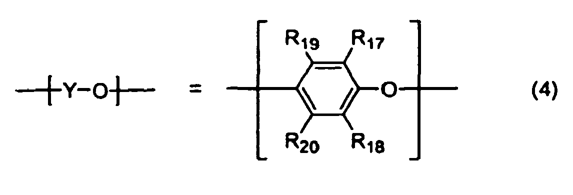 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

2. The bismaleimide according to claim 1, wherein -(O-X-O)- is a structure of the formula (5), the formula (6) or the formula (7) and -(Y-O)- is an arrangement of a structure of the formula (8) or the formula (9) or a random arrangement of structures of the formula (8) and the formula (9), 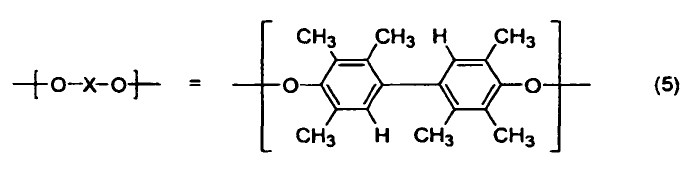 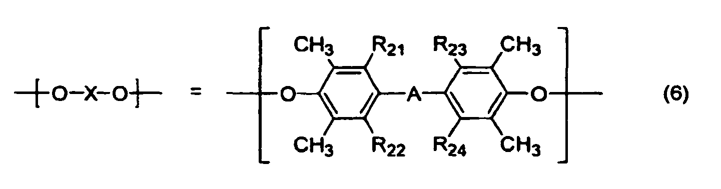 wherein R₂₁, R₂₁, R₂₃ and R₂₄ are the same or different and represent a hydrogen atom or a methyl group, and -A- is a linear, branched or cyclic bivalent hydrocarbon group having 20 or less carbon atoms, 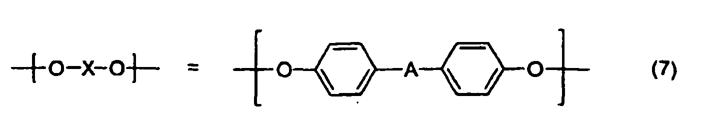 wherein -A- is a linear, branched or cyclic bivalent hydrocarbon group having 20 or less carbon atoms.

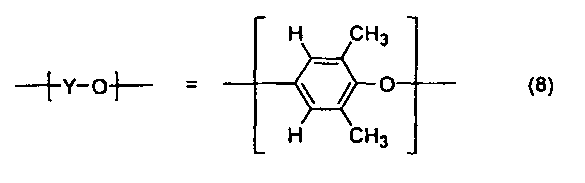

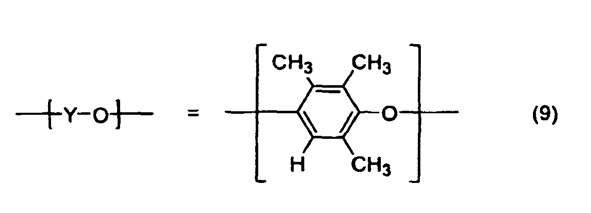

3. A bismaleamic acid represented by the formula (10), 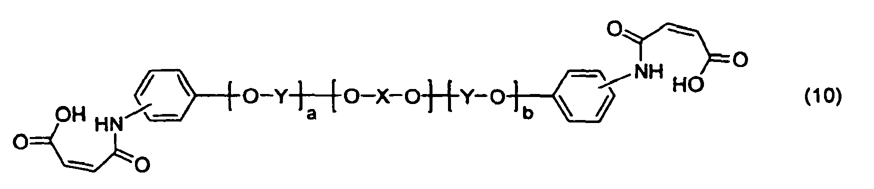 wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each amide group is a para-position or a meta-position, 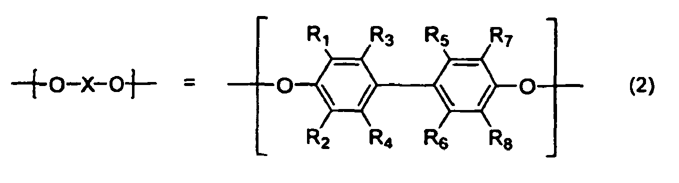 wherein R₁, R₂, R₃, R₇ and R₈ are the same or different and represent a halogen atom, an alkyl group having 6 or less carbon atoms or a phenyl group, R₄, R₅ and R₆ are the same or different and represent a hydrogen atom, a halogen atom, an alkyl group having 6 or less carbon atoms or a phenyl group, 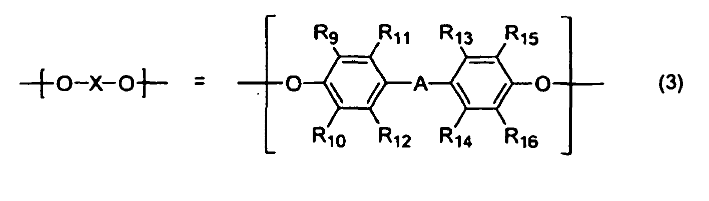 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- is a linear, branched or cyclic bivalent hydrocarbon group having 20 or less carbon atoms, 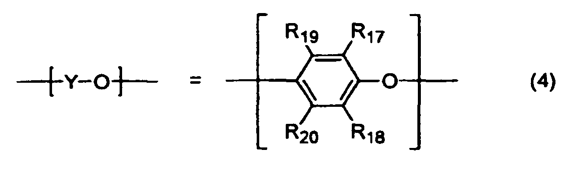 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

4. The bismaleamic acid according to claim 3, wherein -(O-X-O)- is a structure of the formula (5), the formula (6) or the formula (7) and -(Y-O)- is an arrangement of a structure of the formula (8) or the formula (9) or a random arrangement of structures of the formula (8) and the formula (9), 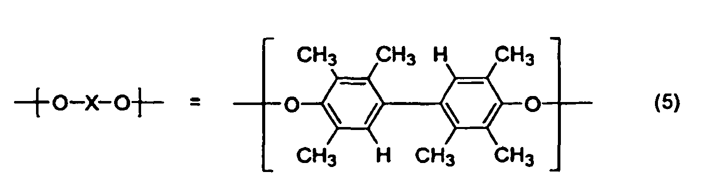 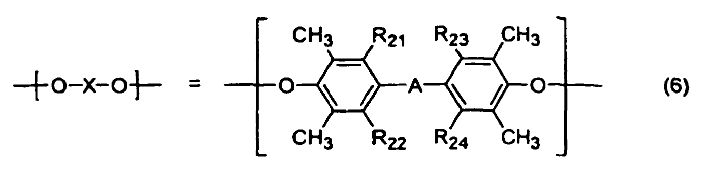 wherein R₂₁, R₂₂, R₂₃ and R₂₄ are the same or different and represent a hydrogen atom or a methyl group and -A- is a linear, branched or cyclic bivalent hydrocarbon group having 20 or less carbon atoms, 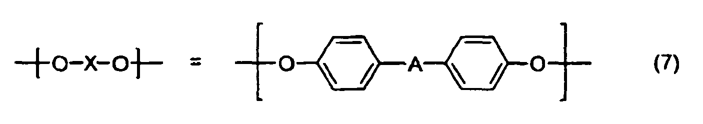 wherein -A- is a linear, branched or cyclic bivalent hydrocarbon group having 20 or less carbon atoms.

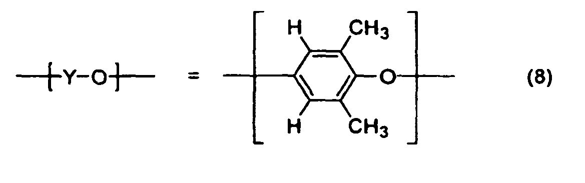

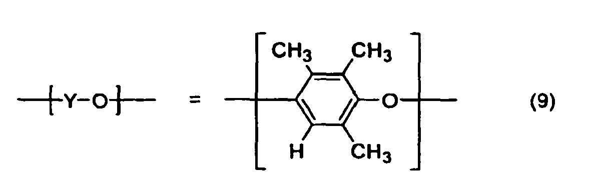

5. A process for the production of the bismaleamic acid represented by the formula (10) as defined in claim 3, which process comprises reacting a diamine represented by the formula (11) with maleic anhydride, 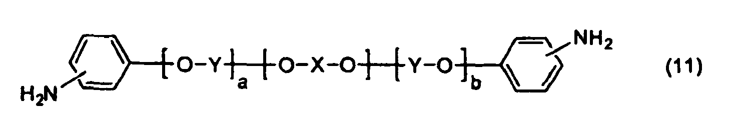 wherein -(O-X-O)- is a structure of the formula (2) or the formula (3), -(Y-O)- is an arrangement of one kind of structure of the formula (4) or a random arrangement of at least two kinds of structures of the formula (4), each of a and b is an integer of 0 to 100, provided that at least one of a and b is not 0, and a substitution position of each amine group is a para-position or a meta-position, 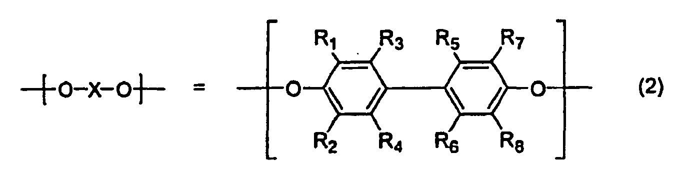 wherein R₁, R₂, R₃, R₇ and R₈ are the same or different and represent a halogen atom, an alkyl group having 6 or less carbon atoms or a phenyl group, R₄, R₅ and R₆ are the same or different and represent a hydrogen atom, a halogen atom, an alkyl group having 6 or less carbon atoms or a phenyl group, 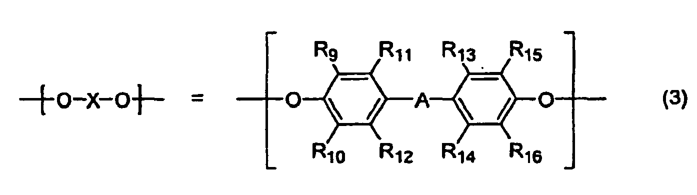 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- is a linear, branched or cyclic bivalent hydrocarbon group having 20 or less carbon atoms, 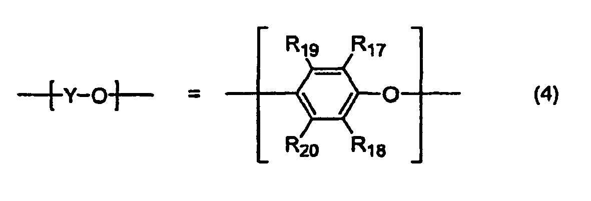 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

6. A process for the production of the bismaleimide represented by the formula (1) as defined in claim 1, which process comprises cyclodehydrating and imidizing the bismaleamic acid represented by the formula (10) as defined in claim 3.

7. A curable resin composition containing at least one kind of bismaleimide represented by the formula (1) as defined in claim 1.

8. A cured product obtained by curing at least one kind of bismaleimide represented by the formula (1) as defined in claim 1.

## Patentansprüche

1. Bismaleimid, dargestellt durch die Formel (1), 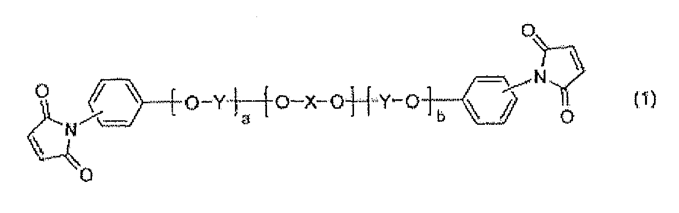 wobei -(O-X-O)- eine Struktur der Formel (2) oder Formel (3) ist, -(Y-O)- eine Anordnung von einer Art von Struktur der Formel (4) oder eine Zufallsanordnung von wenigstens zwei Arten von Strukturen der Formel (4) ist, wobei a und b jeweils eine ganze Zahl von 0 bis 100 ist, unter der Voraussetzung, dass wenigstens eines von a und b nicht gleich 0 ist, und wobei eine Substitutionsposition jeder Imidgruppe eine para-Position oder eine meta-Position ist, 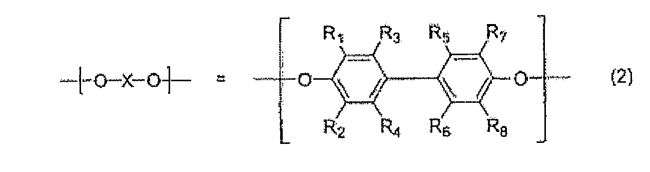 wobei R₁, R₂, R₃, R₇ und R₈ dieselben oder unterschiedlich sind und ein Halogenatom, eine Alkylgruppe mit 6 oder weniger Kohlenstoffatomen oder eine Phenylgruppe darstellen, R₄, R₅ und R₆ dieselben oder unterschiedlich sind und ein Wasserstoffatom, ein Halogenatom, eine Alkylgruppe mit 6 oder weniger Kohlenstoffatomen oder eine Phenylgruppe darstellen, 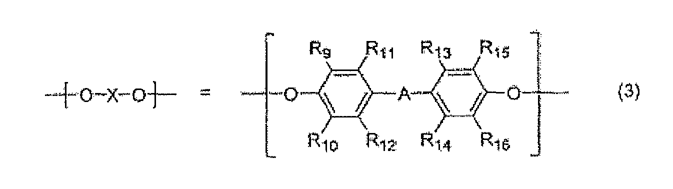 wobei R₉, R₁₀, R₁₁, R₁₂, R₁₃, R₁₄, R₁₅ und R₁₆ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen ist, 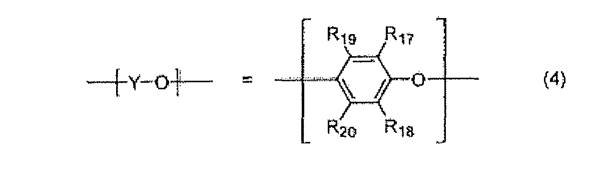 wobei R₁₇ und R₁₈ dieselben oder unterschiedlich sind und ein Halogenatom, eine Alkylgruppe mit 6 oder weniger Kohlenstoffatomen oder eine Phenylgruppe darstellen, und R₁₉ und R₂₀ dieselben oder unterschiedlich sind und ein Wasserstoffatom, ein Halogenatom, eine Alkylgruppe mit 6 oder weniger Kohlenstoffatomen oder eine Phenylgruppe darstellen.

2. Bismaleimid nach Anspruch 1, wobei -(O-X-O)- eine Struktur der Formel (5), der Formel (6) oder der Formel (7) ist, und wobei -(Y-O)- eine Anordnung einer Struktur der Formel (8) oder der Formel (9) oder eine Zufallsanordnung der Strukturen der Formel (8) und der Formel (9) sind, 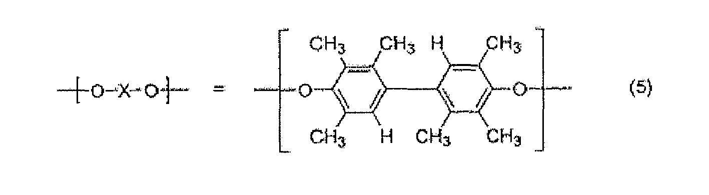 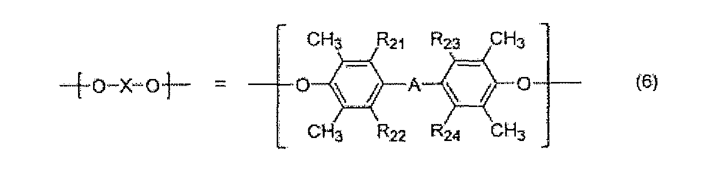 wobei R₂₁, R₂₂, R₂₃ und R₂₄ dieselben oder unterschiedlich sind und ein Wasserstoffatom oder eine Methylgruppe darstellen, und wobei -A- eine lineare, verzweigte oder zyklisch bivalente Kohlenwasserstoffgruppe mit 20 oder weniger Kohlenstoffatomen ist, 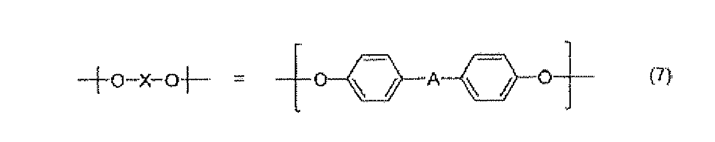 wobei -A- eine lineare, verzweigte oder zyklische bivalente Kohlenwasserstoffgruppe mit 20 oder weniger Kohlenstoffatomen ist,

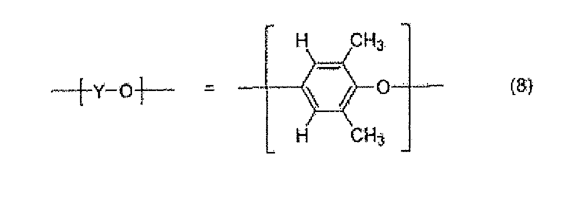

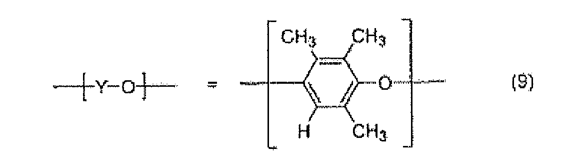

3. Bismaleaminsäure, dargestellt dargestellt durch die Formel (10), 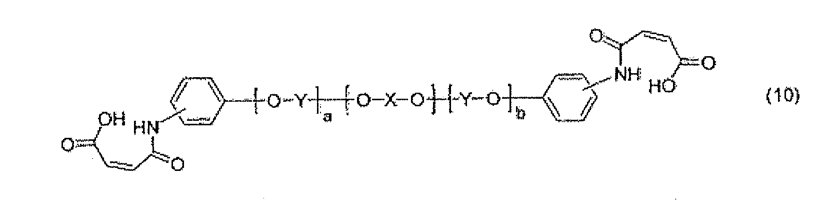 wobei -(O-X-O)- eine Struktur der Formel (2) oder der Formel (3) ist, -(Y-O)- eine Anordnung von einer Art von Struktur der Formel (4) oder eine Zufallsanordnung von wenigsten zwei Arten von Strukturen der Formel (4) ist, a und b jeweils eine ganze Zahl von 0 bis 100 ist, unter der Voraussetzung, dass wenigstens eines von a und b nicht gleich 0 ist, und wobei eine Substitutionsposition jeder Amingruppe eine para-Position oder eine meta-Position ist, 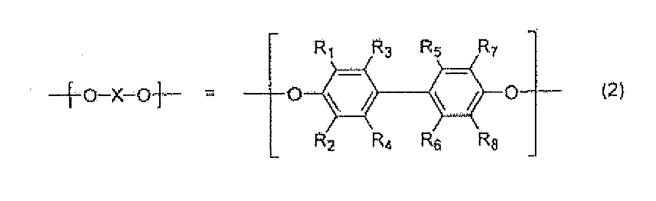 wobei R₁, R₂, R₃, R₇ und R₈ dieselben oder unterschiedlich sind und ein Halogenatom, eine Alkylgruppe mit 6 oder weniger Kohlenstoffatomen oder eine Phenylgruppe darstellen, R₄, R₅ und R₆ dieselben oder unterschiedlich sind und ein Wasserstoffatom, ein Halogenatom , eine Alkylgruppe mit 6 oder weniger Kohlenstoffatomen oder eine Phenylgruppe darstellen, 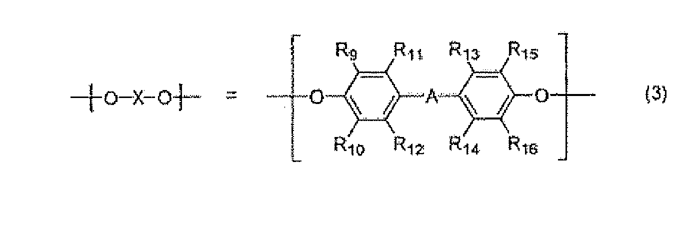 wobei R₉, R₁₀, R₁₂, R₁₃, R₁₄, R₁₅ und R₁₆ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen ist, 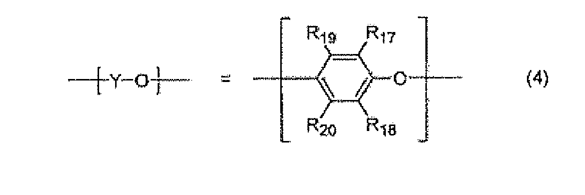 wobei R₁₇ und R₁₈ dieselben oder unterschiedlich sind und ein Halogenatom, eine Alkylgruppe mit 6 oder weniger Kohlenstoffatomen oder eine Phenylgruppe darstellen, und R₁₉ and R₂₀ dieselben oder unterschiedlich sind und ein Wasserstoffatom, ein Halogenatom, eine Alkylgruppe mit 6 oder weniger Kohlenstoffatomen oder eine Phenylgruppe darstellen.

4. Bismaleaminsäure nach Anspruch 3, wobei -(O-X-O)- eine Struktur der Formel (5), der Formel (6) oder der Formel (7) ist, und wobei -(Y-O)- eine Anordnung einer Struktur der Formel (8) oder der Formel (9) oder eine Zufallsanordnung von Strukturen der Formel (8) und der Formel (9) ist, 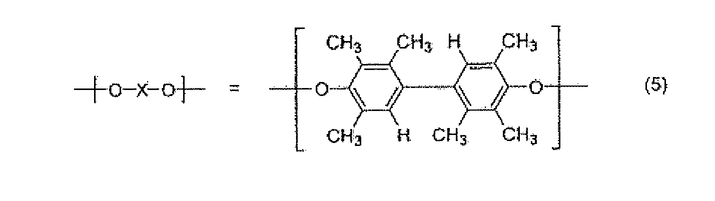 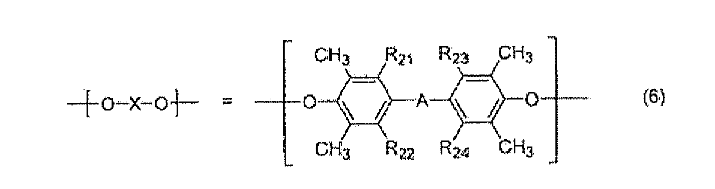 wobei R₂₁, R₂₂, R₂₃ und R₂₄ dieselben oder unterschiedlich sind und ein Wasserstoffatom oder eine Methylgruppe darstellen, und wobei -A- eine lineare, verzweigte oder zyklische bivalente Kohlenwasserstoffgruppe mit 20 oder weniger Kohlenstoffatomen ist, 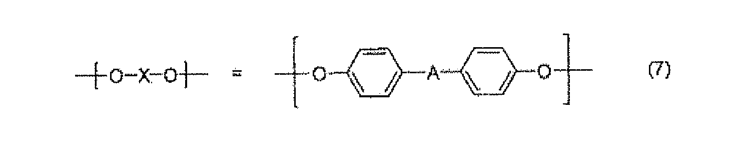 wobei -A- eine lineare, verzweigte oder zyklische bivalente Kohlenwasserstoffgruppe mit 20 oder weniger Kohlenstoffatomen ist,

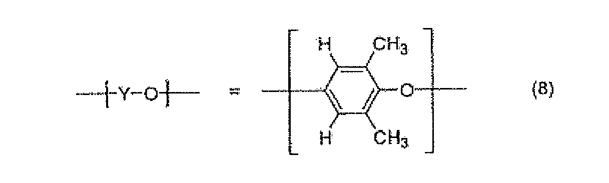

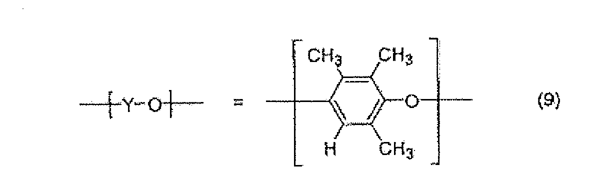

5. Verfahren zur Herstellung der Bismaleaminsäure, dargestellt durch die Formel (10), wie in Anspruch 3 definiert, wobei das Verfahren umfasst:
Umsetzen eines Diamins, dargestellt durch die Formel (11), mit Maleinsäureanhydrid, 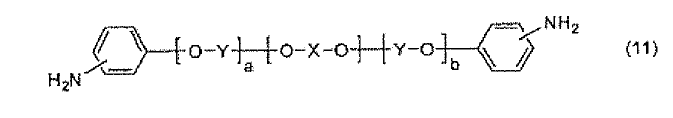 wobei -(O-X-O)- eine Struktur der Formel (2) oder der Formel (3) ist, -(Y-O)- eine Anordnung von einer Art von Struktur der Formel (4) oder eine Zufallsanordnung von mindestens zwei Arten von Strukturen der Formel (4) ist, a und b jeweils eine ganze Zahl von 0 bis 100 ist, unter der Voraussetzung, dass wenigsten eines von a und b nicht gleich 0 ist, und wobei eine Substitutionsposition jeder Aminogruppe eine para-Position oder eine meta-Position ist, 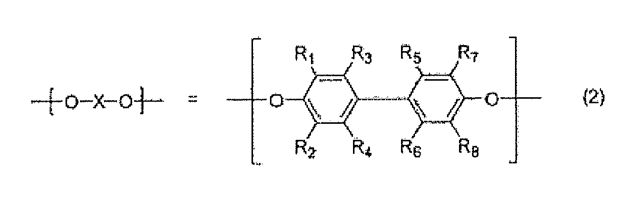 wobei R₁, R₂, R₃, R₇ und R₈ dieselben oder unterschiedlich sind und ein Halogenatom, eine Alkylgruppe mit 6 oder weniger Kohlenstoffatomen oder eine Phenylgruppe darstellen, R₄, R₅ und R₆ dieselben oder unterschiedlich sind und ein Wasserstoffatom, ein Halogenatom, eine Alkylgruppe mit 6 oder weniger Kohlenstoffatomen oder eine Phenylgruppe darstellen, 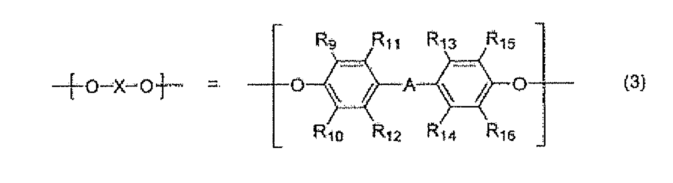 wobei R₉, R₁₀, R₁₁, R₁₂, R₁₃, R₁₄, R₁₅ und R₁₆ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen ist, 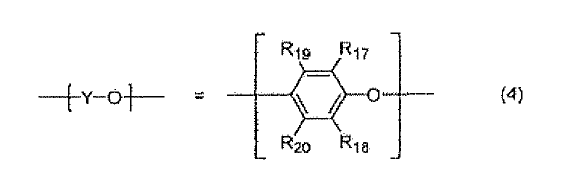 wobei R₁₇ und R₁₈ dieselben oder unterschiedlich sind und ein Halogenatom, eine Alkylgruppe mit 6 oder weniger Kohlenstoffatomen oder eine Phenylgruppe darstellen, und R₁₉ und R₂₀ dieselben oder unterschiedlich sind und ein Wasserstoffatom, ein Halogenatom, eine Alkylgruppe mit 6 oder weniger Kohlenstoffatomen oder eine Phenylgruppe darstellen.

6. Verfahren zur Herstellung des Bismaleimids, dargestellt durch die Formel (1), wie in Anspruch 1 definiert, wobei das Verfahren das Zyklodehydrieren und Imidieren der Bismaleaminsäure, dargestellt durch die Formel (10), wie in Anspruch 3 definiert, umfasst.

7. Härtbare Harzzusammensetzung, umfassend wenigstens eine Art von Bismaleimid, dargestellt durch die Formel (1), wie in Anspruch 1 definiert.

8. Gehärtetes Produkt, erhalten durch Härten von wenigstens einer Art von Bismaleimid, dargestellt durch die Formel (1), wie in Anspruch 1 definiert.

## Revendications

1. Bismaléimide représenté par la formule (1), 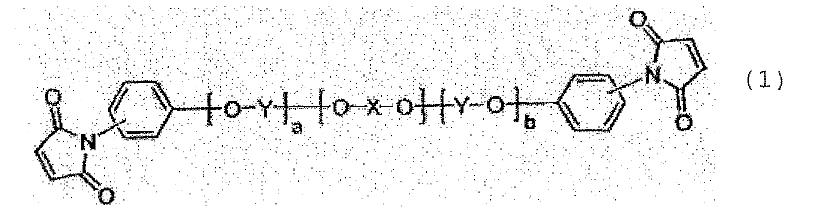 dans laquelle -(O-X-O)- représente une structure de formule (2) ou de formule (3), -(Y-O)- représente un assemblage d'une structure du type de la formule (4) ou un assemblage aléatoire d'au moins deux structures du type de la formule (4), chacun des a et b représente un entier allant de 0 à 100, à condition qu'au moins un des a et b ne représente pas 0, et qu'une position de substitution de chaque groupe imide soit une position para ou une position méta, 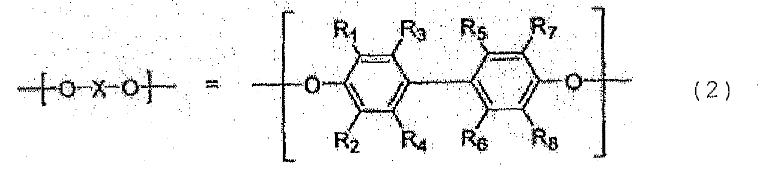 dans laquelle R₁, R₂, R₃, R₇ et R₈ sont identiques ou différents et représentent un atome d'halogène, un groupe alkyle ayant 6 atomes de carbone ou moins ou un groupe phényle, R₄, R₅ et R₆ sont identiques ou différents et représentent un atome d'hydrogène, un atome d'halogène, un groupe alkyle ayant 6 atomes de carbone ou moins ou un groupe phényle, 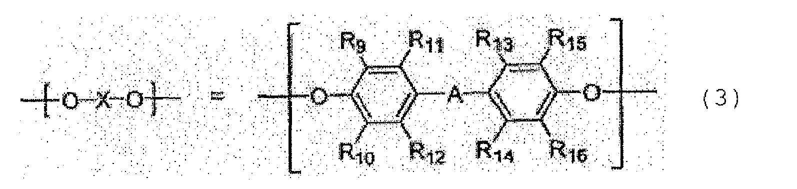 dans laquelle R₉, R₁₀, R₁₁, R₁₂, R₁₃, R₁₄, R₁₅ et R₁₆ sont identiques ou différents et représentent un atome d'hydrogène, un atome d'halogène, un groupe alkyle ayant 6 atomes de carbone ou moins ou un groupe phényle, et -A- représente un groupe hydrocarboné divalent alinéaire, ramifié ou cyclique ayant 20 atomes de carbone ou moins, 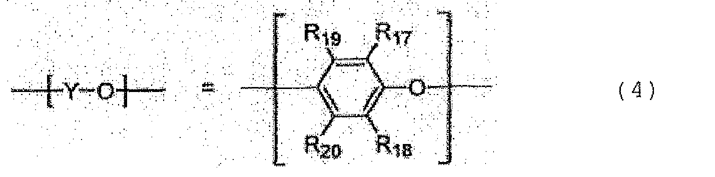 dans laquelle R₁₇ et R₁₈ sont identiques ou différents et représentent un atome d'halogène, un groupe alkyle ayant 6 atomes de carbone ou moins, ou un groupe phényle, et R₁₉ et R₂₀ sont identiques ou différents et représentent un atome d'hydrogène, un atome d'halogène, un groupe alkyle ayant 6 atomes de carbone ou moins, ou un groupe phényle.

2. Bismaléimide selon la revendication 1, dans lequel -(O-X-O)- représente une structure de formule (5), de formule (6) ou de formule (7), et -(Y-O)- représente un assemblage d'une structure de formule (8) ou de formule (9) ou un assemblage aléatoire de structures de formule (8) et de formule (9), 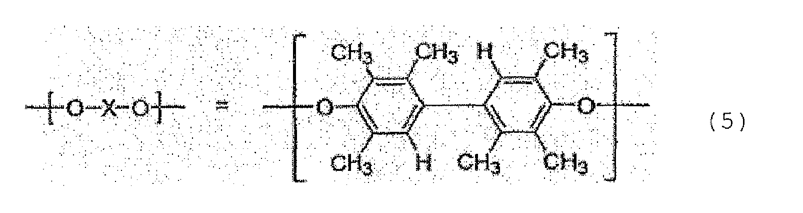 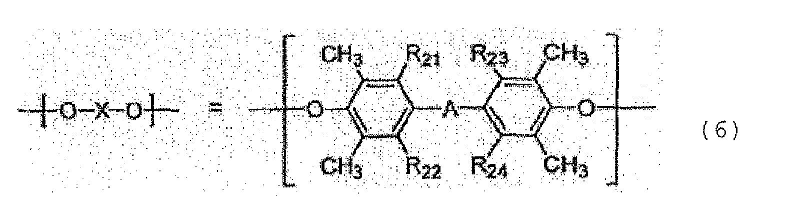 dans laquelle R₂₁, R₂₂, R₂₃ et R₂₄ sont identiques ou différents et représentent un atome d'hydrogène ou un groupe méthyle, et -A- représente un groupe hydrocarboné divalent linéaire, ramifié ou cyclique ayant 20 atomes de carbone ou moins, 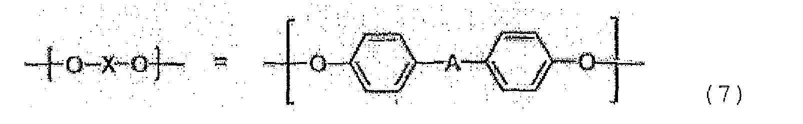 dans laquelle -A- représente un groupe hydrocarboné divalent linéaire, ramifié ou cyclique ayant 20 atomes de carbone ou moins.

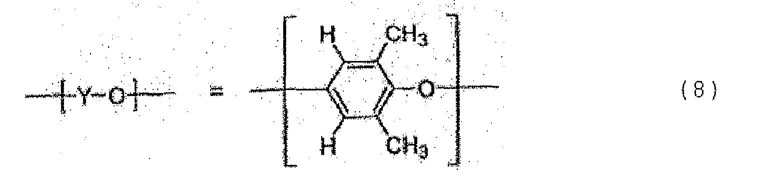

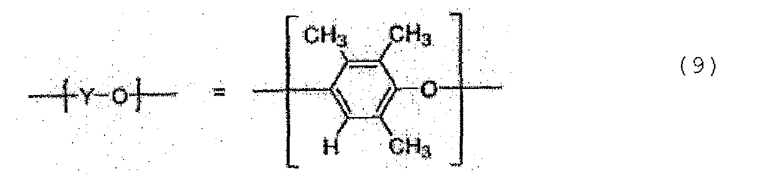

3. Acide bismaléamique représenté par la formule (10), 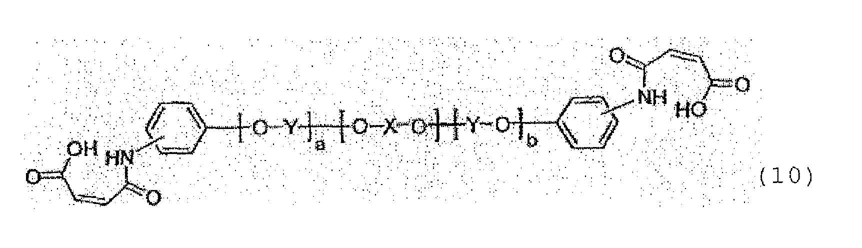 dans laquelle -(O-X-O)- représente une structure de formule (2) ou de formule (3), -(Y-O)- représente un assemblage d'une structure du type de la formule (4) ou un assemblage aléatoire d'au moins deux structures du type de la formule (4), chacun des a et b représente un entier allant de 0 à 100, à condition qu'au moins un des a et b ne représente pas 0, et qu'une position de substitution de chaque groupe amine soit une position para ou une position méta, 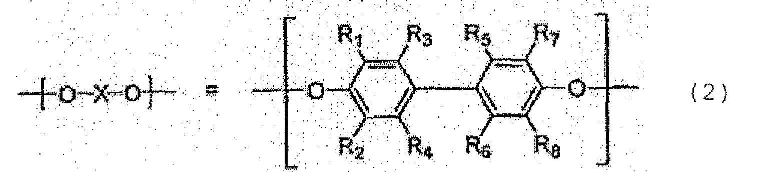 dans laquelle R₁, R₂, R₃, R₇ et R₈ sont identiques ou différents et représentent un atome d'halogène, un groupe alkyle ayant 6 atomes de carbone ou moins ou un groupe phényle, R₄, R₅ et R₆ sont identiques ou différents et représentent un atome d'hydrogène, un atome d'halogène, un groupe alkyle ayant 6 atomes de carbone ou moins ou un groupe phényle, 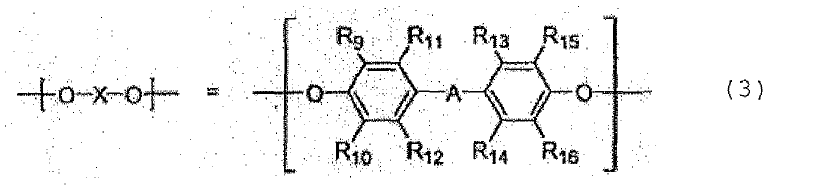 dans laquelle R₉, R₁₀, R₁₁, R₁₂, R₁₃, R₁₄, R₁₅ et R₁₆ sont identiques ou différents et représentent un atome d'hydrogène, un atome d'halogène, un groupe alkyle ayant 6 atomes de carbone ou moins ou un groupe phényle, et -A- représente un groupe hydrocarboné divalent linéaire, ramifié ou cyclique ayant 20 atomes de carbone ou moins, 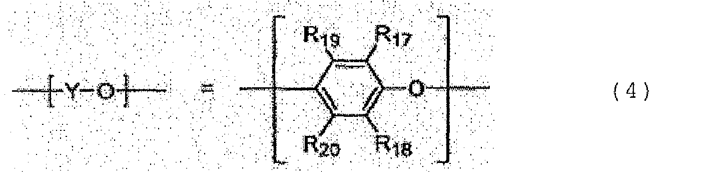 dans laquelle R₁₇ et R₁₈ sont identiques ou différents et représentent un atome d'halogène, un groupe alkyle ayant 6 atomes de carbone ou moins ou un groupe phényle, et R₁₉ et R₂₀ sont identiques ou différents et représentent un atome d'hydrogéne, un atome d'halogène, un groupe alkyle ayant 6 atomes de carbone ou moins ou un groupe phényle.

4. Acide bismaléamique selon la revendication 3, dans lequel -(O-X-O)- représente une structure de formule (5), de formule (6) ou de formule (7), et -(Y-O)- représente un assemblage d'une structure de formule (8) ou de formule (9) ou un assemblage aléatoire de structures de formule (8) et de formule (9), 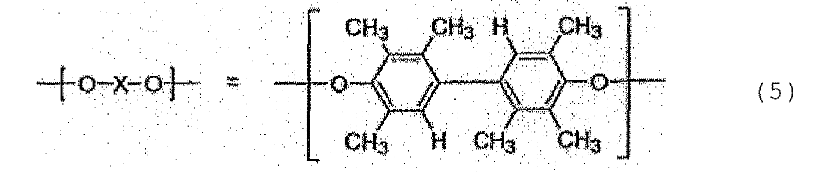 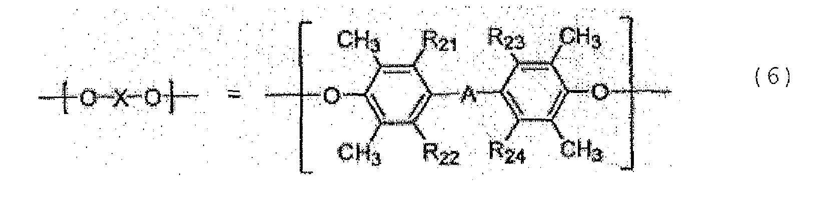 dans laquelle R₂₁, R₂₂, R₂₃ et R₂₄ sont identiques ou différents et représentent un atome d'hydrogène ou un groupe méthyle, et -A- représente un groupe hydrocarboné divalent linéaire, ramifié ou cyclique ayant 20 atomes de carbone ou moins, 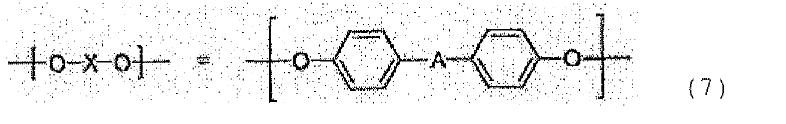 dans laquelle -A- représente un groupe hydrocarboné divalent linéaire, ramifié ou cyclique ayant 20 atomes de carbone ou moins.

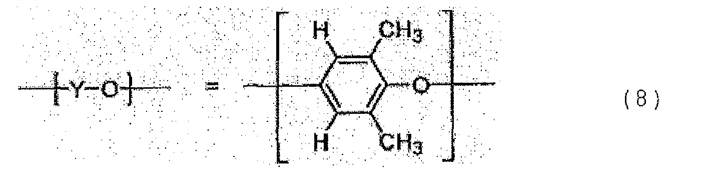

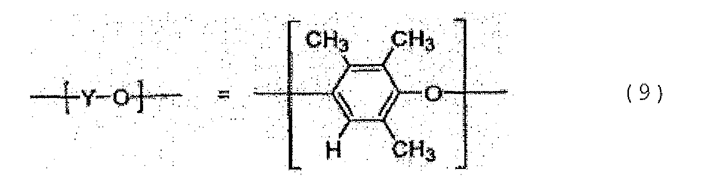

5. Procédé de production de l'acide bismaléamique représenté par la formule (10) telle que définie dans la revendication 3, ledit procédé comprenant la réaction d'une diamine représentée par la formule (11) avec l'anhydride maléique, 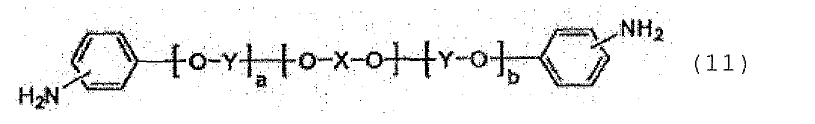 dans laquelle -(O-X-O)- représente une structure de formule (2) ou de formule (3), -(Y-O)-représente un assemblage d'une structure du type de la formule (4) ou un assemblage aléatoire d'au moins deux structures du type de la formule (4), chacun des a et b représente un entier allant de 0 à 100, à condition qu'au moins un des a et b ne représente pas 0, et qu'une position de substitution de chaque groupe amine soit une position para ou une position méta, 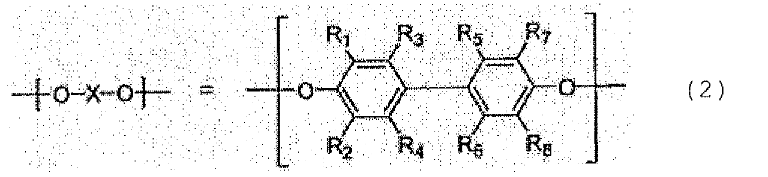 dans laquelle R₁, R₂, R₃, R₇ et R₈ sont identiques ou différents et représentent un atome d'halogène, un groupe alkyle ayant 6 atomes de carbone ou moins ou un groupe phényle, R₄, R₅ et R₆ sont identiques ou différents et représentent un atome d'hydrogène, un atome d'halogène, un groupe alkyle ayant 6 atomes de carbone ou moins ou un groupe phényle, 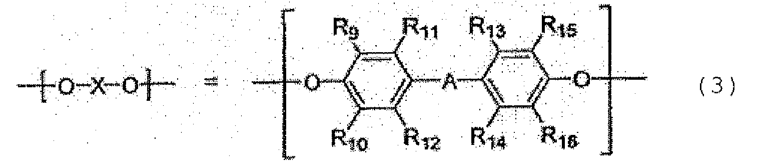 dans laquelle R₉, R₁₀, R₁₁, R₁₂, R₁₃, R₁₄, R₁₅ et R₁₆ sont identiques ou différents et représentent un atome d'hydrogène, un atome d'halogène, un groupe alkyle ayant 6 atomes de carbone ou moins ou un groupe phényle, et -A- représente un groupe hydrocarboné divalent linéaire, ramifié ou cyclique ayant 20 atomes de carbone ou moins, 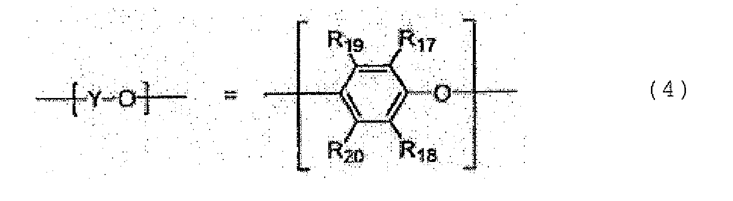 dans laquelle R₁₇ et R₁₈ sont identiques ou différents et représentent un atome d'halogène, un groupe alkyle ayant 6 atomes de carbone ou moins ou un groupe phényle, et R₁₉ et R₂₀ sont identiques ou différents et représentent un atome d'hydrogène, un atome d'halogène, un groupe alkyle ayant 6 atomes de carbone ou moins ou un groupe phényle.

6. Procédé de production du bismaléimide représenté par la formule (1) tel que défini dans la revendication 1, ledit procédé comprenant la cyclodéshydratation et l'imidation de l'acide bismaléamique représenté par la formule (10) telle que définie dans la revendication 3.

7. Composition de résine durcissable contenant au moins un type de bismaleimide représenté par la formule (1) telle que définie dans la revendication 1.

8. Produit durci obtenu en durcissant au moins un type de bismaleimide représenté par la formule (1) telle que définie dans la revendication 1.
